# EUROPEAN PATENT SPECIFICATION

(11) **EP 3 500 859 B1**
(45) Date of publication and mention of the grant of the patent: **08.12.2021**
(21) Application number: 17754708.0
(22) Date of filing: 17.08.2017
(51) Int. Cl.: G01N 33/569, G01N 33/50, A61K 38/17, A61K 35/12, C07K 14/705, C12N 5/07, G01N 15/00

(54) **MARKER FOR NEURAL STEM CELLS**
MARKER FÜR NEURALE STAMMZELLEN
MARQUEUR POUR CELLULES SOUCHES NEURALES

(30) Priority: 17.08.2016 SE 1651107
(43) Date of publication of application: 26.06.2019
(73) Proprietor: Xintela AB, 223 81 Lund (SE)
(72) Inventor: LUNDGREN ÅKERLUND, Evy, 237 33 Bjärred (SE); CHMIELARSKA MASOUMI, Katarzyna, 224 67 Lund (SE)
(74) Representative: Høiberg P/S
(86) International application number: PCT/EP2017/070838
(87) International publication number: WO 2018/033596

(56) References cited:
- WO-A1-03/106492
- WO-A1-2007/099337
- WO-A2-02/086082
- SIMONA CASAROSA ET AL: "Neural stem cells: ready for therapeutic applications?", MOLECULAR AND CELLULAR THERAPIES, BIOMED CENTRAL LTD, LONDON, UK, vol. 2, no. 1, 15 October 2014 (2014-10-15), page 31, XP021201374, ISSN: 2052-8426, DOI: 10.1186/2052-8426-2-31
- SEUNG U. KIM ET AL: "Neural stem cell-based treatment for neurodegenerative diseases : NSCs for neurodegenerative diseases", NEUROPATHOLOGY, vol. 33, 1 February 2013 (2013-02-01), pages 491-504, XP055414635, JP ISSN: 0919-6544, DOI: 10.1111/neup.12020
- PETER E. HALL ET AL: "Integrins Are Markers of Human Neural Stem Cells", STEM CELLS., vol. 24, no. 9, 1 September 2006 (2006-09-01), pages 2078-2084, XP055413805, US ISSN: 1066-5099, DOI: 10.1634/stemcells.2005-0595 cited in the application
- BERGSTRÖM TOBIAS ET AL: "Developmentally regulated collagen/integrin interactions confer adhesive properties to early postnatal neural stem cells", BIOCHIMICA ET BIOPHYSICA ACTA (BBA) - GENERAL SUBJECTS, vol. 1840, no. 8, 23 January 2014 (2014-01-23), pages 2526-2532, XP028872920, ISSN: 0304-4165, DOI: 10.1016/J.BBAGEN.2014.01.021
- LISA A. FLANAGAN ET AL: "Regulation of human neural precursor cells by laminin and integrins", JOURNAL OF NEUROSCIENCE RESEARCH., vol. 83, no. 5, 1 January 2006 (2006-01-01), pages 845-856, XP055413798, US ISSN: 0360-4012, DOI: 10.1002/jnr.20778 cited in the application
- MATTHEW B MAAS ET AL: "Molecular biomarkers in stroke diagnosis and prognosis", BIOMARKERS IN MEDICINE, vol. 3, no. 4, 1 August 2009 (2009-08-01), pages 363-383, XP055415015, UK ISSN: 1752-0363, DOI: 10.2217/bmm.09.30
- Quynh Vu et al.: "Meta-analysis of preclinical studies of mesenchymal stromal cells for ischemic stroke", Neurology, vol. 82, no. 14 8 April 2014 (2014-04-08), pages 1277-1286, XP055328174, DOI: 10.1212/WNL.0000000000000278 Retrieved from the Internet: URL:http://n.neurology.org/content/82/14/1 277 [retrieved on 2016-12-12]
- ARIANNA BENVENUTO ET AL: "Pharmacotherapy of autism spectrum disorders", BRAIN AND DEVELOPMENT, vol. 35, no. 2, 1 February 2013 (2013-02-01), pages 119-127, XP055437850, NL ISSN: 0387-7604, DOI: 10.1016/j.braindev.2012.03.015
- XIAOHUA LI ET AL: "Review of Pharmacological Treatment in Mood Disorders and Future Directions for Drug Development", NEUROPSYCHOPHARMACOLOGY., vol. 37, no. 1, 7 September 2011 (2011-09-07), pages 77-101, XP055437826, US ISSN: 0893-133X, DOI: 10.1038/npp.2011.198
- SÁRA KÁLMÁN ET AL: "A Dishful of a Troubled Mind: Induced Pluripotent Stem Cells in Psychiatric Research", STEM CELLS INTERNATIONAL, vol. 2016, 29 December 2015 (2015-12-29), pages 1-21, XP055437819, US ISSN: 1687-966X, DOI: 10.1155/2016/7909176 cited in the application

## Description

### Technical field

The present disclosure relates to a marker for identification and isolation of mammalian neural stem cells and neural progenitor cells, as well as uses thereof for preparing enriched cellular populations of neural stem cells and neural progenitor cells. The disclosure further relates to use of neural stem cells, neural progenitor cells and mesenchymal stem cells for treating disease and damage, as well as preventing and protecting from damage of the nervous system. Furthermore the disclosure relates to use the marker to detect and diagnose the damage and as a prognostic marker.

### Background

The complex, delicate structures that make up the nervous system ― the brain, spinal cord and peripheral nerves ― are susceptible to various types of injury ranging from trauma to neurodegenerative diseases that cause progressive deterioration. Unfortunately, very little spontaneous regeneration, repair or healing occurs after injuries. Therefore, brain damage, paralysis due to spinal cord injury and peripheral nerve damage are often permanent and incapacitating. Patients with serious nervous system injuries or stroke often require lifelong assistance. Stroke is among the most frequent causes of death and adult disability, especially in highly developed countries. However, treatment options to date are very limited. Innovative, new strategies are thus required to advance treatment of neurological injury and stroke.

Neural stem cells (NSCs) are cells in the central nervous system (CNS) with the capacity to proliferate, self-renew and generate a large number of progenitors of both neurons and glia. During the process of adult neurogenesis, NSCs undergo numerous stages, including NSCs self-renewal, transient amplifying progenitors, neuroblasts, and terminally mature neurons, astrocytes, and oligodendrocytes (Gage F. H. and Temple S., 2013). NSCs have been identified in nearly all regions of the embryonic mouse, rat and human CNS. In the adult CNS, neural stem cells and neural progenitor cells have been shown to contribute to neurogenesis in specialized stem cell niches. NSCs are thus useful for regeneration of nervous tissue following disease and damage.

Neural stem cells (NSC) and progenitor cells (NPC) have the ability to form all the major cell types of the central nervous system making them candidates for cell-based therapies in brain injuries and neurodegenerative disorders. However, two reasons have hampered the development of cell therapies for clinical applications. One is the difficulty to isolate NSCs from human tissues and expand the cells in sufficient quantities for therapy. The other is the inability to purify the NSCs from more differentiated cell types and other contaminating cells. Specific cellular markers of neural stem cells/neural progenitor cells (NSC) are therefore critical for the identification, isolation and selection of human NSCs for therapeutic applications.

Several markers have been used over the years to identify different cell types and differentiation stages:
Sox2 (SRY box 2), a member of the Sox family of transcription factors, and Nestin, an intracellular filament protein, are frequently utilized as markers of NSCs in both the embryonic and adult brains. However, since both markers are found intracellularly, they cannot readily be used for isolating and selecting functional NSCs.
PSA-NCAM, polysialylated neural cell adhesion molecule, is highly expressed in neural progenitor cells during brain development. However, it is also found in differentiated neural cells (Kim HS et al. 2014; Butenschön J et al. 2016).
GFAP, glial fibrillary acidic protein, an intermediate filament protein, is found on neural progenitor cells, but is also primarily seen as the major intermediate filament protein in mature astrocytes (Zhang QB et al. 2006).
CD133 (prominin-1) is present in different types of stem cells including NSCs, but is also expressed on differentiated cells (Kania G, Corbeil D, Fuchs J et al. 2005; Zhang QB et al. 2006).
PDGFR-α is found throughout the adult CNS and is evenly distributed along the ventricular wall (Chojnacki et al. 2011). Within the adult subventricular zone (SVZ), PDGFR-α has been found to label a specific population of cells that are type B neural stem cells (Jackson et al. 2006) and that generate primarily oligodendrocytes (Chojnacki et al. 2011). Studies suggest that PDGFR-α regulates the balance between neuronal and oligodendrocytic production (Farahani and Xaymardan 2015). PDGFR-α is thus expressed by some differentiated neural cells but is mainly found on oligodendrocyte progenitors.

Musashi-1 (Msi-1), an RNA-binding protein, is putatively expressed in CNS stem and progenitor cells where it regulates proliferation and maintenance (for a review, see MacNicol et al. 2008). However, it is localised in the cytoplasm and nucleus of cells, i.e. intracellularly, and can thus not readily be used for isolating and selecting functional NSCs.

CD24 is a cell adhesion molecule that is found on immune cells and cells of the CNS. In the brain, CD24 is found within neurogenic zones in the young adult mouse and co-localizes with PSA-NCAM, hence being recognized as a neuroblast (type A NSC) marker (Calaora et al. 1996). It has been used to isolate NSCs from the mouse brain by flow cytometry (Rietze et al. 2001; Panchision et al. 2009) and in combination with other cell surface markers (such as CD15 and CD29) is used to enrich neuronal cultures (Pruszak et al. 2009). However, since levels of CD24 increase upon maturation of neuronal cells, it is also considered a marker of early neuronal differentiation (Pruszak et al. 2009).

LeX, also known as SSEA-1 or CD15 is a marker of immature neuroepithelial cells lining the ventricles in the adult CNS and of differentiating postmitotic neurons (Calaora et al. 1996; Capela and Temple, 2002). While its expression overlaps with GFAP, only few (4%) isolated cells from the SVZ are LeX-positive. The fact that LeX is shed by SVZ cells in the extracellular niche can explain such low levels of free LeX. Furthermore, LeX is expressed by both types B and C cells of the adult mouse SVZ, but not type A (neuroblasts) cells (Capela and Temple, 2006).

Vimentin is one of the main intermediate filament proteins that is mainly expressed by radial-glia and immature astrocytes during early brain development.

β-III tubulin or Tuj1 is an intracellular filament marker of immature neurons. It is involved in axon guidance and maintenance.

Further, WO 02/086082 relates to markers for neural stem cells and discloses cell cultures of neural stem or progenitor cells that have been isolated using gangliosides as markers.

Finally, some members of the integrin family have been suggested as markers of human neural stem cells, including some integrins of the β1 subfamily which consists of 12 different integrins where different α-subunits are combined with integrin β1 to form unique receptors with different functions. The integrin subunit β1 has been used to select NSC from fetal brain tissue, however, selection based on the β1 subunit is not specific because integrins in the β1 subfamily are present on most cells including differentiated cells. It is unclear which of the α-integhns on NSCs that partners with β1. Expression of α2, α3, α5, α6 and α7 has all been detected on NSCs (Flanagan et al., 2006) but these integrins are also present on a variety of cell types. Hall PE et al. (2006) suggested using the dimer integrin α6β1 as a possible marker of human NSCs. This integrin is a receptor for vascular laminins and known to play a role in platelet adhesion, activation and arterial thrombosis.

In conclusion, there is an unmet need for specific markers suitable for the identification and isolation of neural stem cells and neural progenitor cells.

### Summary

The integrin α10β1 is a collagen type II binding receptor found on chondrocytes (Camper et al., 1998). Integrin α10β1 is a major collagen-binding integrin on chondrocytes that it is highly expressed in cartilage, both during development and in adult tissues (Camper et al., 2001). Integrin α10β1 is also expressed by mesenchymal stem cells (MSCs) (Varas et al., 2007). It has furthermore been shown that fibroblast growth factor -2 (FGF-2) upregulates expression of integrin α10β1 and improves chondrogenic potential of MSCs (Varas et al., 2007). Bengtsson et al (2005) demonstrated that mice lacking the integrin α10β1 have defects in the cartilaginous growth plate and, as a consequence, develop growth retardation of the long bones. A recent study revealed that a naturally occurring mutation in the canine α10 integrin gene is responsible for chondrodysplasia in hunting dog breeds (Kyöostilä et al., 2013), supporting a critical role for α10β1 in skeletal development.

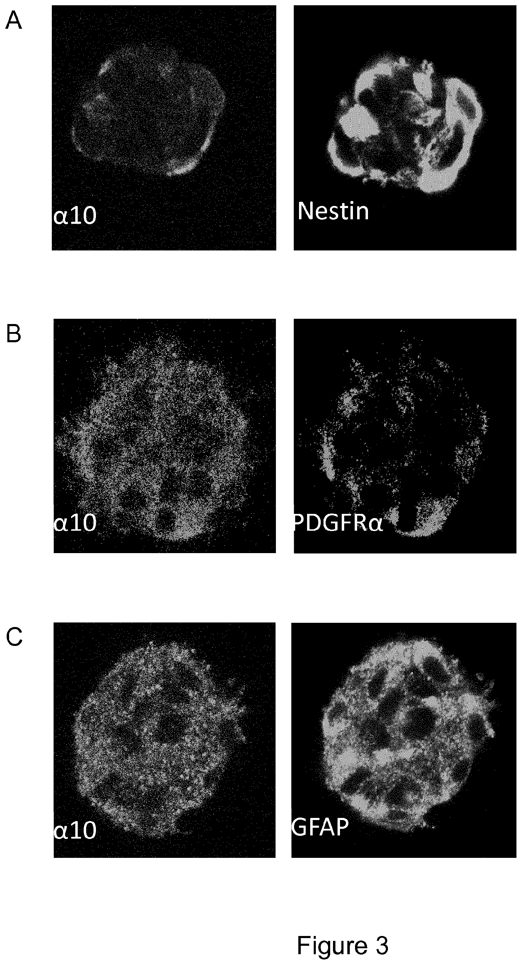

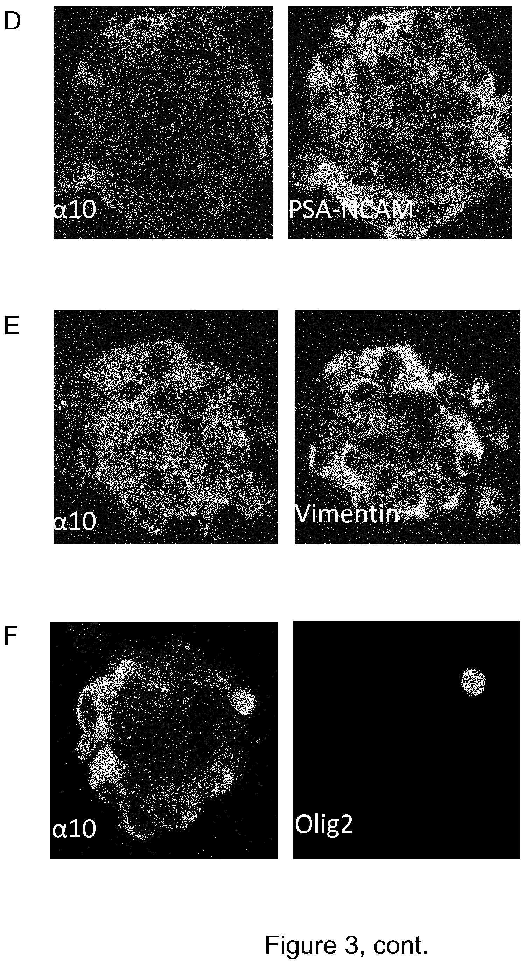

The present inventors have surprisingly detected expression of integrin heterodimer α10β1 on neural stem cells and neural progenitor cells derived from neural tissue, and disclose herein its use as a selective marker for identification and isolation of mammalian neural stem cells and neural progenitor cells. In contrast to the above-mentioned NSC surface markers, integrin α10β1 is present on all three NSC/NPC cell types of the adult mouse SVZ stem cell niche (see table 1 below) as shown by colocalisation studies (Figures3, 6, 7, 8, 9,10, 11 and 12) suggesting that integrin α10β1 is a broader stem and progenitor cell marker, compared to other known markers, covering the different subtypes of the brain stem cell niche.

**Table 1. Cell types of the adult mouse SVZ identified by NSC surface markers. Type A: neuroblasts, which will give rise to mature neurons; Type B: stem cells, astroglial cells - adjacent to a layer of ependymal cells (E cells); Type C: transit amplifying cells.**

| **Surface marker** | **SVZ cell type** | | |
|---|---|---|---|
| | A | B | C |
| PDGFR-α | | ✔ | |
| CD24 | ✔ | | |
| LeX | | ✔ | ✔ |
| Integrin α10β1 | ✔ | ✔ | ✔ |

One aspect of the present disclosure relates to the use of a marker comprising an integrin alpha 10 subunit expressed by a neural stem cell and/or a neural progenitor cell as a marker for mammalian neural stem cells and mammalian neural progenitor cells.

Another aspect of the present disclosure relates to an in vitro method for identifying a mammalian neural stem cell and/or a mammalian neural progenitor cell, the method comprising the steps of:
a) detecting expression of an integrin alpha10 subunit by a cell comprised in a sample comprising neural tissue,
b) scoring the integrin alpha10 subunit expression of b), and
c) identifying the mammalian neural stem cell and/or the neural progenitor cell according to the scoring in b).

Another aspect of the present disclosure relates to an in vitro method for isolating a mammalian neural stem cell and/or a mammalian neural progenitor cell, the method comprising the steps of:
a) detecting expression of an integrin alpha10 subunit by a cell comprised in a sample comprising neural tissue,
b) scoring the integrin alpha10 subunit expression of a), and
c) selecting the mammalian neural stem cell and/or the mammalian neural progenitor cell according to the scoring in b).

Another aspect of the present disclosure relates to a method for determining whether a test compound modulates a mammalian neural stem cell and/or a mammalian neural progenitor cell differentiation in vitro, the method comprising the steps of
a) providing a neural stem cell and/or a neural progenitor cell that expresses integrin alpha10 subunit,
b) contacting the neural stem cell and/or the neural progenitor cell with a test compound, and
c) detecting a change in rate or pattern of differentiation of the neural stem cell and/or neural progenitor cell as an indication of that the test compound modulates a neural stem cell and/or a neural progenitor cell differentiation,
   wherein the rate or pattern of differentiation is determined by detecting integrin alpha10 expression by the cell according to the method described herein.

Another aspect of the present disclosure relates to a method for manufacturing an isolated population of mammalian cells in vitro which are enriched for neural stem cells and/or neural progenitor cells relative to a reference population, the method comprising the steps of
a) providing at least a portion of a population of cells from the CNS, or a portion of a reference population, comprising a neural stem cell and/or a neural progenitor cell,
b) introducing into the population of cells in a) above a compound identifying an integrin alpha10 subunit expressed by the neural stem cell and/or neural progenitor cell,
c) selecting and isolating from the population of cells in b) above the neural stem cells and/or the neural progenitor cells,
   thereby producing a population of cells enriched for neural stem cells and/or neural progenitor cells.

Another aspect of the present disclosure relates to an *in vitro* cell culture of undifferentiated mammalian cells expressing an integrin alpha10 subunit, wherein the cells are derived from neural tissue and wherein
a) cells in the culture have the capacity to differentiate into neurons and/or oligodendrocytes and/or astrocytes when differentiated in a culture medium substantially free of both serum and a proliferation-inducing growth factor;
b) the cell in culture proliferates in a culture medium containing a serum replacement such as B27 and at least one proliferation-inducing growth factor;
c) cells in the culture differentiate into neurons and/or oligodendrocytes and/or astrocytes upon withdrawal of both serum replacement B27 and the proliferation inducing growth factor.

Another aspect of the present disclosure relates to an *in vitro* cell culture comprising
a) a culture medium containing a serum replacement such as B27 and at least one proliferation-inducing growth factor; and
b)undifferentiated mammalian cells derived from the central nervous system of a mammal, wherein at least 30%, e.g. at least 40%, e.g. at least 50%, e.g. at least 60%, e.g. at least 70%, e.g. at least 80%, e.g. at least 90%, e.g. at least 95% of the cells express an integrin alpha10 subunit.

Another aspect of the present disclosure relates to a suspension culture of mammalian undifferentiated cells expressing an integrin alpha10 subunit, wherein said cells are substantially formed into cell aggregates, and wherein the cell aggregates are maintained in a culture medium containing a proliferation-inducing growth factor.

Another aspect of the present disclosure relates to a method of treating disease or damage of the nervous system and/or preventing and protecting from CNS damage in a subject in need thereof, the method comprising:
a) providing a composition comprising an enriched population of mammalian neural stem cells and/or mammalian neural progenitor cells, wherein the cells express an integrin alpha10 subunit;
b) administering a therapeutically effective amount of the isolated population of mammalian neural stem cells and/or neural progenitor cells to the subject,
thereby treating the disease or damage and/or preventing and protecting from damage of the central nervous system.

Another aspect of the present disclosure relates to a method of treating a mental and behavioral disorder in a subject in need thereof, the method comprising:
a) providing a composition comprising an enriched population of mammalian neural stem cells and/or mammalian neural progenitor cells, wherein the cells express an integrin alpha10 subunit;
b) administering a therapeutically effective amount of the isolated population of mammalian neural stem cells and/or neural progenitor cells to the subject,
thereby treating the neurologic disorders with psychiatric symptoms.

Another aspect of the present disclosure relates to a method of treating disease or damage and/ or preventing and protecting from damage of the nervous system in a subject in need thereof, the method comprising:
a) providing a composition comprising an enriched population of mammalian mesenchymal stem cells, wherein the cells express integrin alpha10 subunit;
b) administering a therapeutically effective amount of the isolated population of mammalian mesenchymal stem cells to the subject,
thereby treating the disease or damage and/or preventing and protecting from damage of the central nervous system.

Another aspect of the present disclosure relates to a method of treating a mental and behavioural disorder in a subject in need thereof, the method comprising:
a) providing a composition comprising an enriched population of mammalian mesenchymal stem cells, wherein the cells express integrin alpha10 subunit;
b) administering a therapeutically effective amount of the isolated population of mammalian mesenchymal stem cells to the subject,
thereby treating the neurologic disorders with psychiatric symptoms.

Another aspect of the present disclosure relates to a marker for mammalian neural stem cells and/or mammalian neural progenitor cells, comprising an integrin alpha10 chain subunit expressed by the neural stem cell and/or neural progenitor cells, for use in a method of treating a disease or damage and/or preventing and protecting from damage of the nervous system and/or treating a mental and behavioral disorder.

Another aspect of the present disclosure relates to a composition comprising an isolated population of mammalian neural stem cells and/or mammalian neural progenitor cells expressing an integrin alpha10 subunit for use in a method of treatment of disease or damage of the nervous system and/or mental and behavioral disorder.

### Description of Drawings

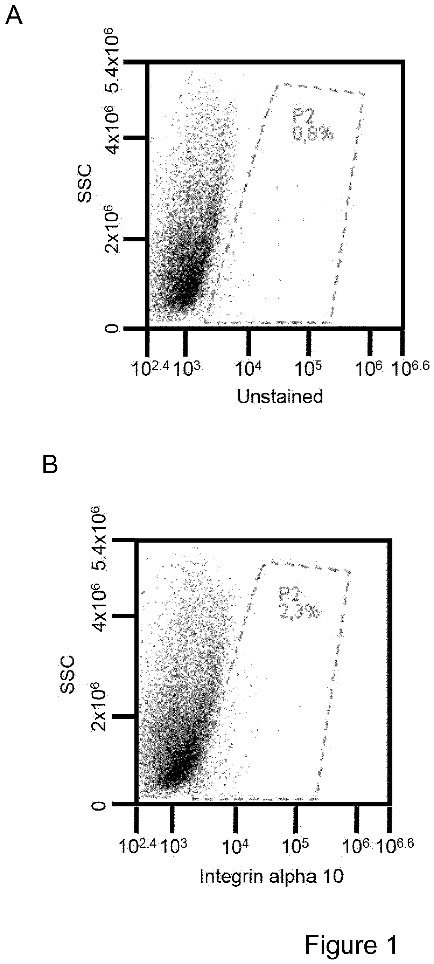

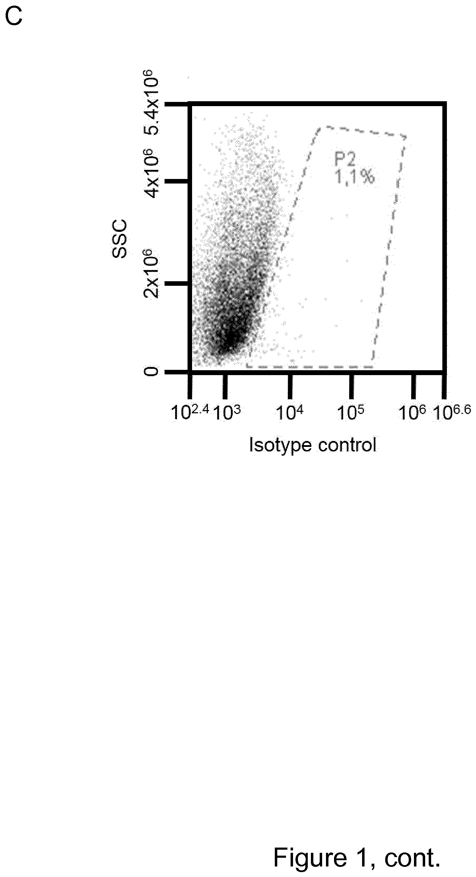

Figure 1. Integrin α10β1 is expressed by a subpopulation of cells isolated from the subventricular zone of adult mouse brain.
   The figure shows expression of integrin α10β1 on cells isolated from the subventricular zone of the adult mouse brain, as assessed by flow cytometry. Analysis of unstained cells (A). Cells stained with a monoclonal antibody directed to the integrin alpha10 subunit (B) and with a control mouse IgG2a antibody (C) and the percentage of positive cells were subsequently analyzed. The results show that a subpopulation of the isolated cells expresses integrin α10β1.

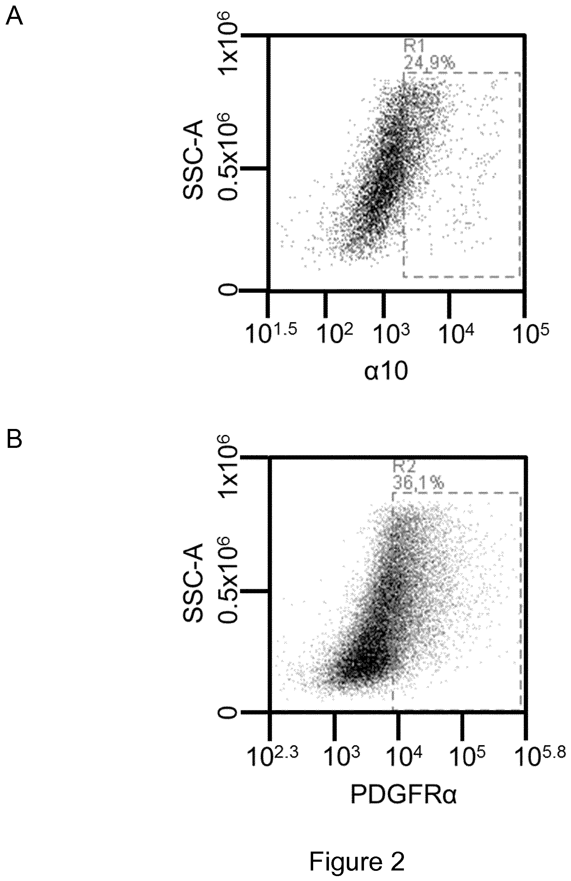

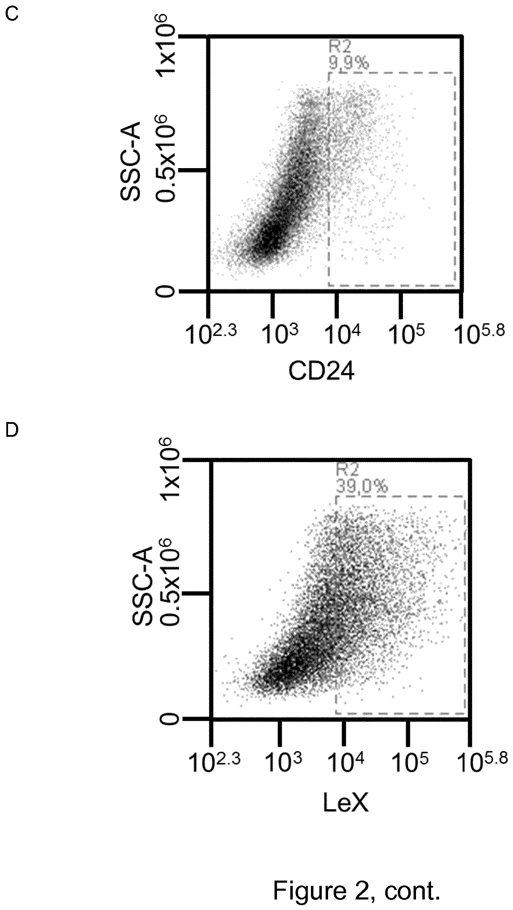

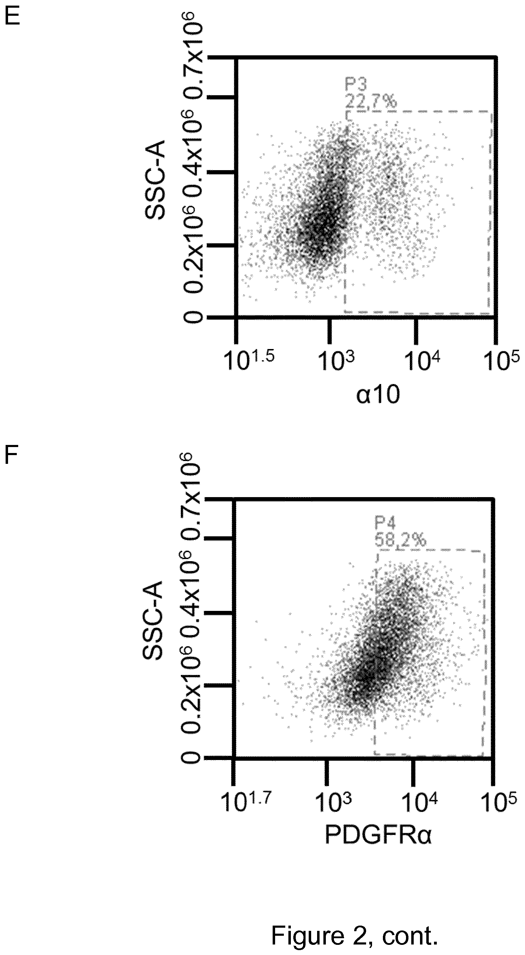

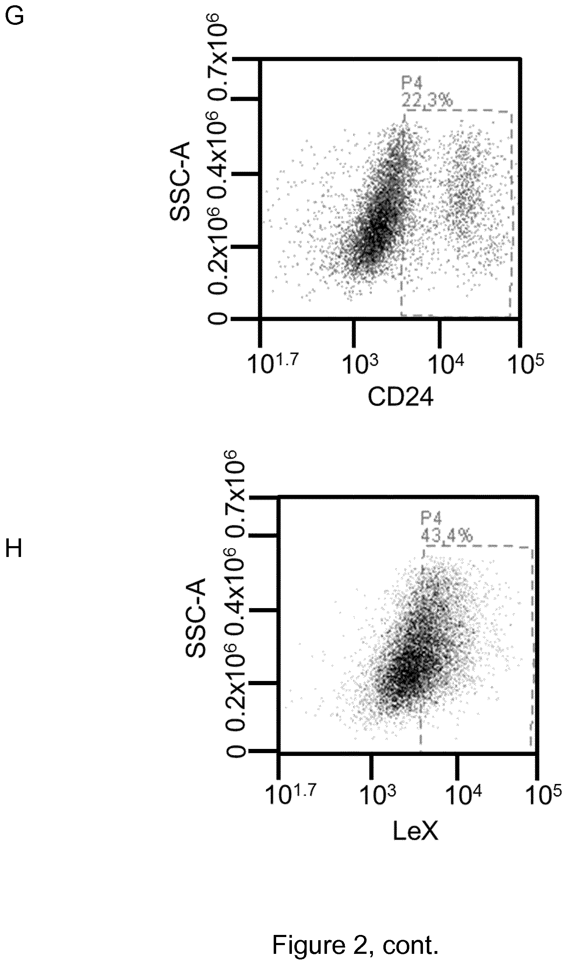

Figure 2. Integrin α10β1 is expressed by a subpopulation of cells isolated from the subventricular zone and cultured as neurospheres or as a monolayer.
   The figure shows expression of integrin α10β1, PDFGRα, Lex and CD24 by cells cultured as a monolayer (A-D) and neurospheres (E-H) as assessed by flow cytometry. Cells were stained with a monoclonal antibody directed to the integrin alpha10 subunit (A, E), a monoclonal antibody directed to PDFGRα (B, F), an antibody directed to CD24 (C,G) and an antibody directed to Lex (D,H) and the percentage of positive cells was analyzed by flow cytometry. The results show that a subpopulation of the cells cultured in monolayer or as neurospheres expresses integrin α10β1, PDGFRα, Lex and CD24.
Figure 3. Neurospheres express integrin α10β1 and other neural stem/progenitor stem cell markers.
   The figure shows the double immunolabeling of cells in neurospheres, isolated from the SVZ of mice and immunostained for integrin α10β1 and other neural stem/progenitor cell markers. Stainings were visualized and images acquired by confocal microscopy. Whole neurosphere shows expression of both α10 and neural stem/progenitor cell markers Nestin (A), PDGFRα (B), GFAP (C), PSA-NCAM (D), Vimentin (E). In agreement with the cellular composition of the SVZ in vivo (Doetsch et al. 1997), neurospheres in vitro also show low abundance of type C cells, as demonstrated by Olig2 staining (F).
Figure 4. Neural stem/progenitor cells isolated from the SVZ and cultured under stem cell conditions retain their potential to differentiate into neuronal and glial cells. Neural stem/progenitor cells were differentiated and expression for neuronal (*Map2, β*- *III Tub*) and glial (*Gfap, O4*) genes were measured. *Gapdh* was used as a housekeeping gene for normalization. Gene expression is expressed as mean ± standard error of the mean (SEM) of triplicate samples.
Figure 5. Integrin α10β1 is expressed by cells of the subventricular zone (SVZ) of the adult mouse brain.
   The figure shows expression of integrin α10β1 in the SVZ of adult mouse tissue as assessed by immunofluorescence staining and confocal microscopy. Brain tissue was stained with a rabbit polyclonal antibody directed to the integrin alpha10 subunit (A) and DAPI to visualize the cell nucleus DNA (B). A composite image of A and B is shown in C.

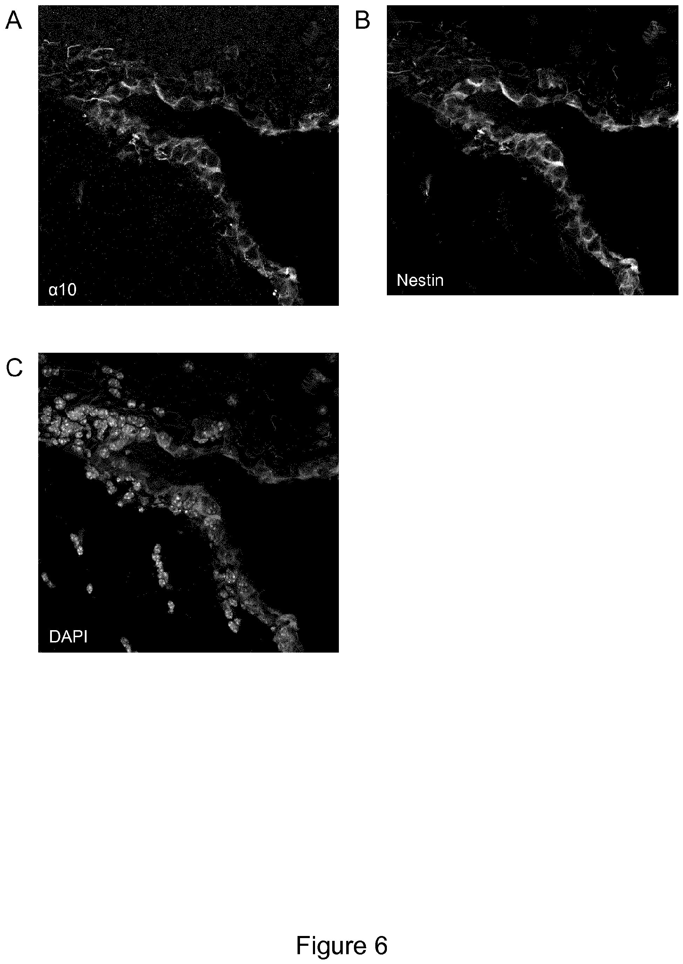

Figure 6. Integrin α10β1 and the neural stem/progenitor cell marker nestin partially co-localize in the subventricular zone (SVZ) of the adult mouse brain.
   The figure shows expression of integrin α10β1 and nestin in the SVZ of adult mouse tissue as assessed by immunofluorescence staining and confocal microscopy. Brain tissue was stained with a rabbit polyclonal antibody directed to the integrin alpha10 subunit (A), a mouse monoclonal antibody directed to nestin (B) and DAPI to visualize the cell nucleus DNA (C).

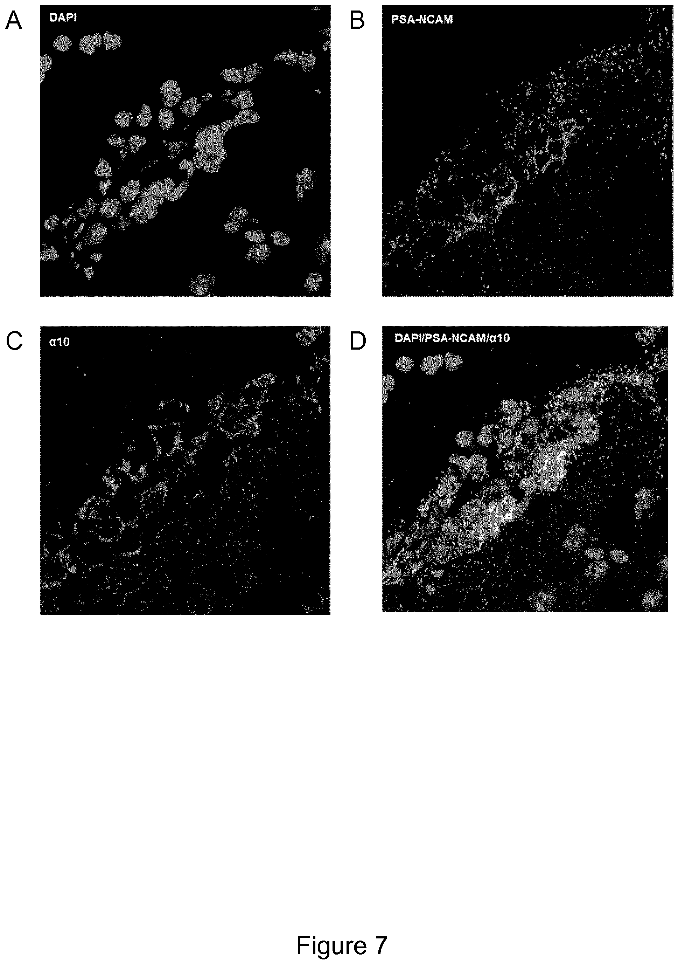

Figure 7. Integrin α10β1 and the neuroblast marker PSA-NCAM partially co-localize on cells in the subventricular zone (SVZ) of the adult mouse brain.
   The figure shows expression of integrin α10β1 and PSA-NCAM in the SVZ of adult mouse brain tissue as assessed by immunofluorescence staining and confocal microscopy. Brain tissue was stained with a rabbit polyclonal antibody directed to the integrin alpha10 subunit (C), a mouse monoclonal antibody directed to PSA-NCAM (B) and DAPI to visualize the cell nucleus DNA (A). A composite image of A, B and C is shown in D.

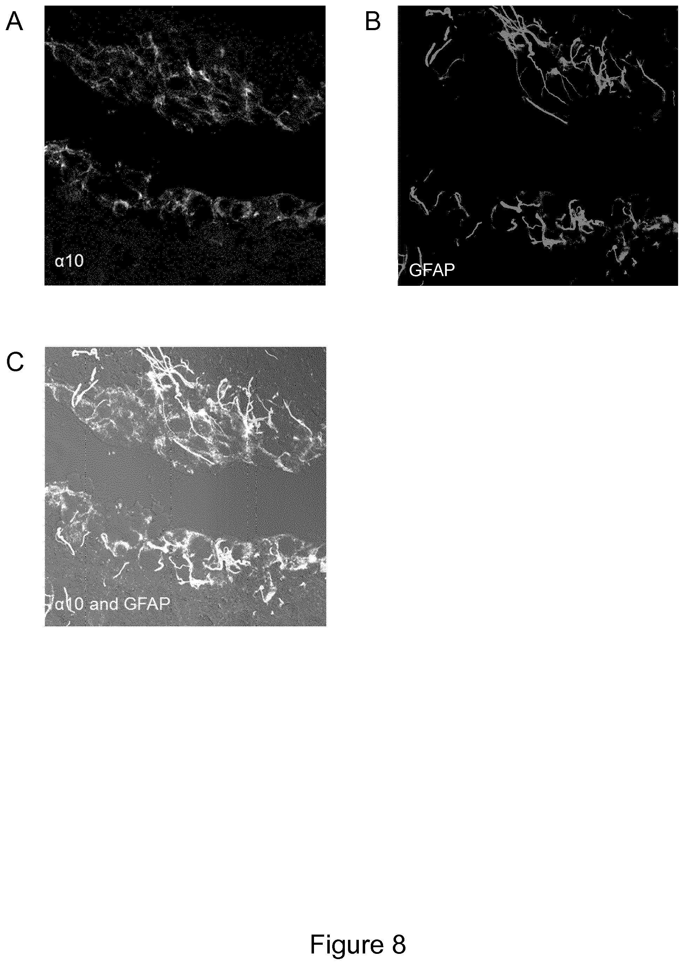

Figure 8. Integrin α10β1 and the glial cells marker GFAP partially co-localize on cells in the subventricular zone (SVZ) in the adult mouse brain.
   The figure shows expression of integrin α10β1 and GFAP in the SVZ of adult mouse brain tissue as assessed by immunofluorescence staining and confocal microscopy. Brain tissue was stained with a rabbit polyclonal antibody directed to the integrin alpha10 subunit (A), a goat polyclonal antibody direct to GFAP (B). A composite image of A and B is shown in C.

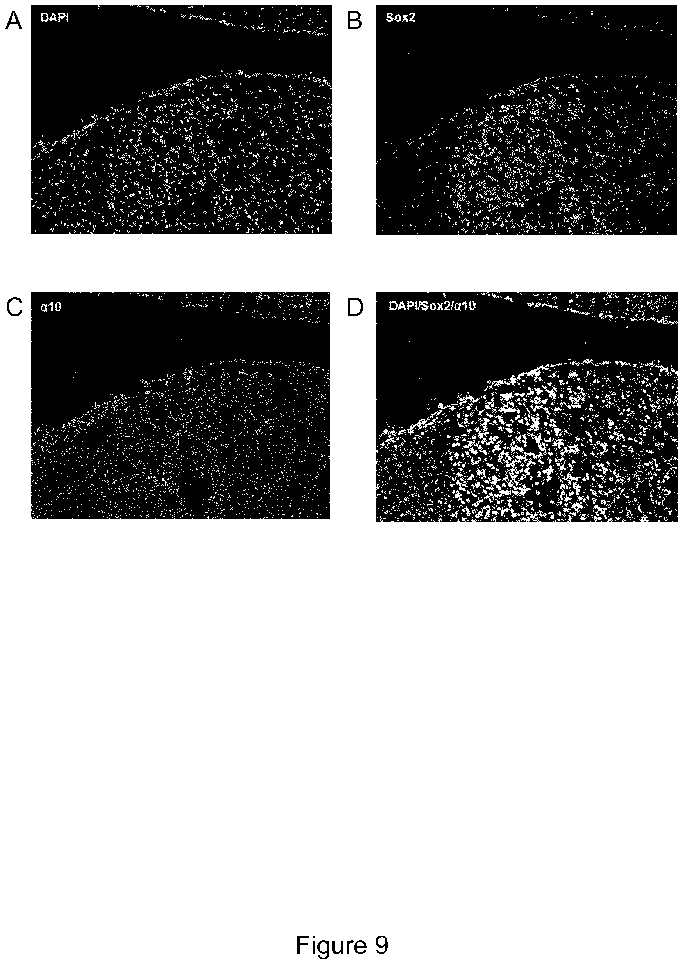

Figure 9. Integrin α10β1 and the neural stem cell marker SOX2 partially co-localize on cells in the subventricular zone (SVZ) in the of newborn mouse brain.
   The figure shows expression of integrin α10β1 and the neural stem cell marker SOX2 in the SVZ of newborn mouse brain tissue as assessed by immunofluorescence staining and epifluorescence microscopy. Brain tissue was stained with DAPI to visualize the cell nucleus DNA (A), a mouse monoclonal antibody direct to SOX2 (B) and a rabbit polyclonal antibody directed to the integrin alpha 10 subunit (C). A composite image of A, B and C is shown in D.

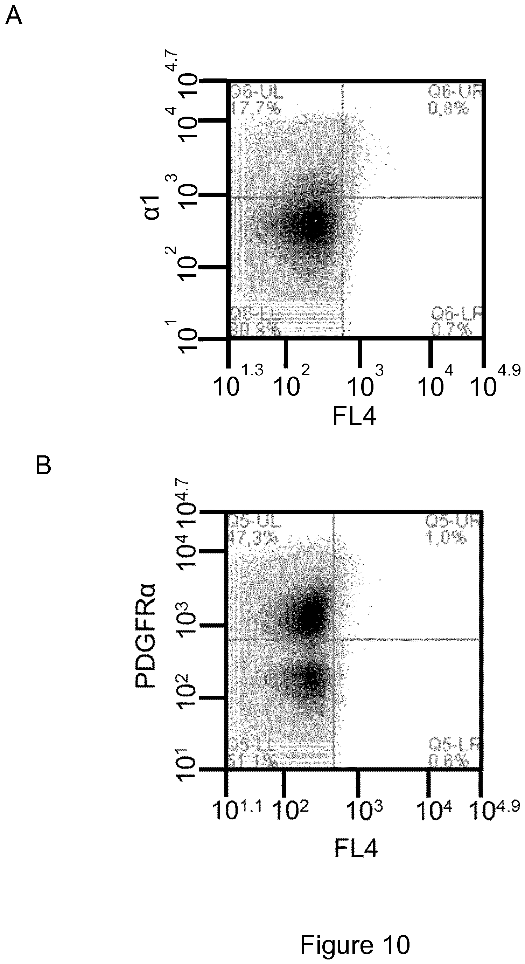

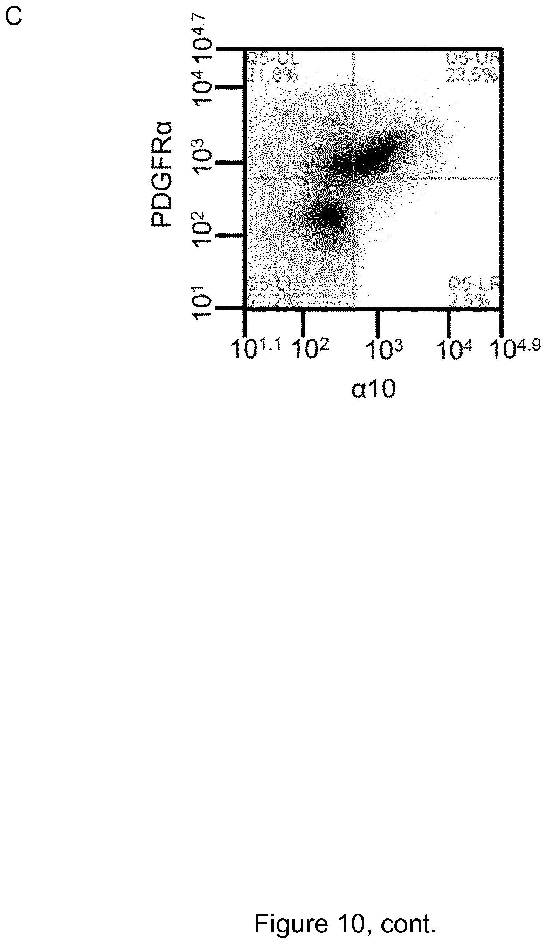

Figure 10. Integrin α10β1 and PDFGRα co-localize on a subpopulation of cells isolated from the subventricular zone in the adult mouse brain.
   The figure shows expression of integrin α10β1 and PDFGRα by cells isolated from the subventricular zone of adult mouse brain as assessed by flow cytometry. Cells were stained with a monoclonal antibody directed to the integrin alpha10 subunit (A) and with a monoclonal antibody directed to PDFGRα (B) and the percentage of positive cells was analyzed.Flow cytometry analysis demonstrates that a subpopulation of the isolated cells expresses both integrin α10β1 and PDFGRα (C).

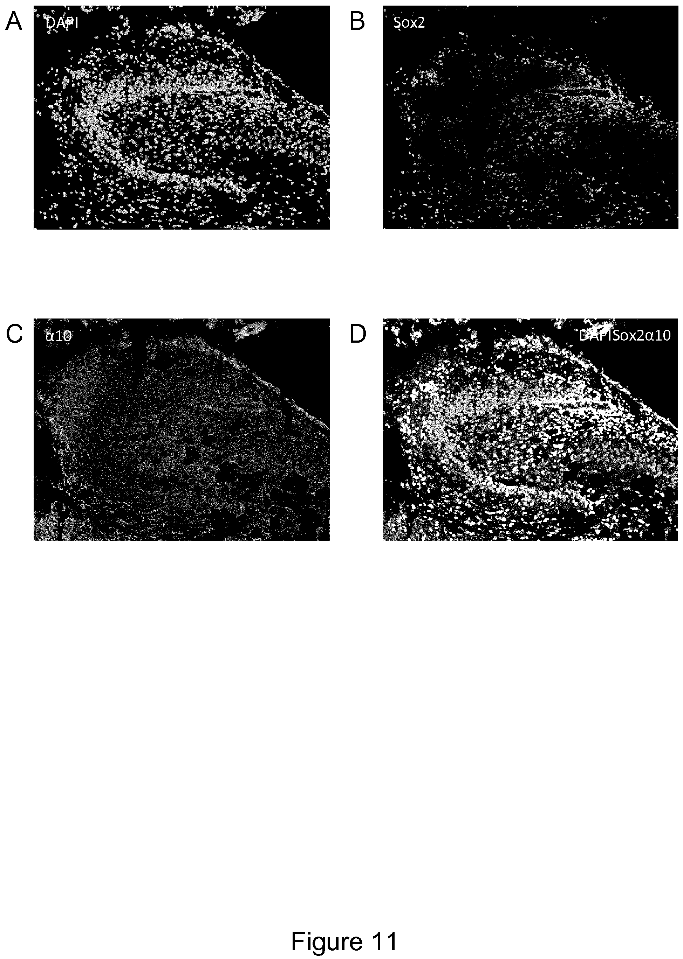

Figure 11. Integrin α10β1 is expressed in the subgranular zone (SGZ) of the hippocampus in the newborn mouse brain and partially co-localizes with the neural stem cell marker SOX2.
   The figure shows expression of integrin α10β1 and neural stem cell marker SOX2 in the SGZ in newborn mouse brain tissue, as assessed by immunofluorescence staining and epifluorescence microscopy. Brain tissue was stained with DAPI to visualize the cell nucleus DNA (A), a mouse monoclonal antibody directed to SOX2 (B) and a rabbit polyclonal antibody directed to the integrin alpha10 subunit (C). A composite image of A, B and C is shown in D.

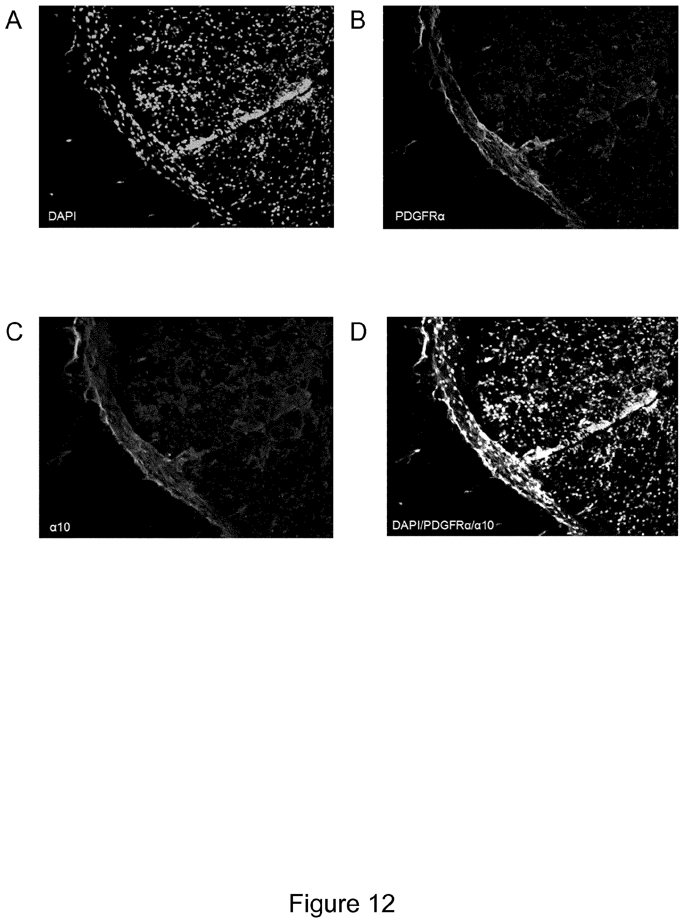

Figure 12. Integrin α10β1 is expressed in the meninges of the newborn mouse brain and partially co-localizes with PDGFRα, a marker of glial cells / oligodendrocyte progenitors.
   The figure shows expression of integrin α10β1 and PDGFRα in the meninges of the newborn mouse brain tissue, as assessed by immunofluorescence staining and epifluorescence microscopy. Brain tissue was stained with DAPI to visualize the cell nucleus DNA (A), a goat polyclonal antibody direct to PDGFRα (B) and a rabbit polyclonal antibody directed to the integrin alpha10 subunit (C). A composite image of A, B and C is shown in D.

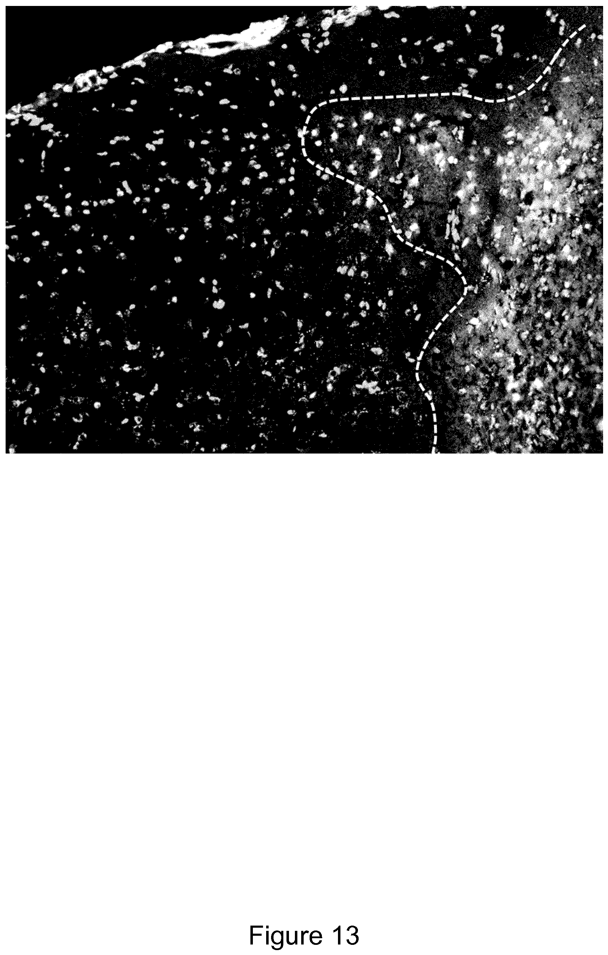

Figure 13. Integrin α10β1 is upregulated in mouse brain following stroke.
   The figure shows higher expression of integrin α10β1 in the stroke area versus intact area in mouse brain, as assessed by immunofluorescence staining and epifluorescence microscopy. The stroke area is the area to the right of the dashed line.

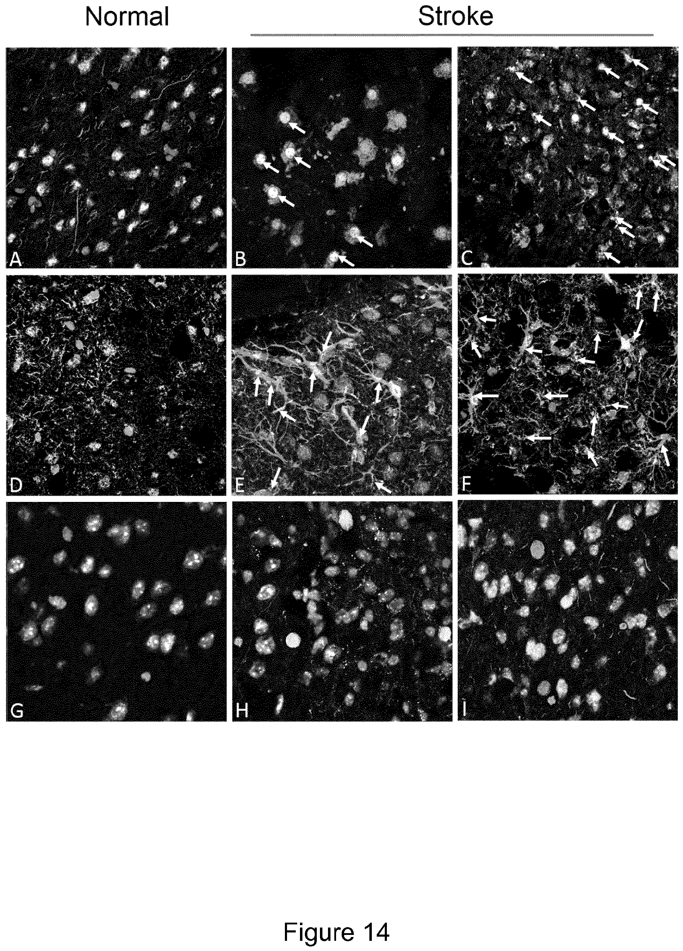

Figure 14. Expression of Integrin α10β1, neuronal marker NeuN, and astrocytic marker GFAP.
   The figure shows expression of integrin α10β1, NeuN, GFAP and Iba1 in the mouse brain assessed by immunofluorescence staining and confocal microscopy. Brain tissue was stained with a rabbit polyclonal antibody directed to the integrin alpha10 subunit, a guinea pig polyclonal antibody direct to NeuN, a goat polyclonal antibodies direct to GFAP and Iba1 (microglial marker). Composite images of control mouse brain is shown in A, D and composite image of stroke brain with regard to expression of integrin α10β1 and NeuN (B and C), expression of integrin α10β1 and GFAP (E and F) and expression of integrin α10β1 and Iba1 (H and I), Colocalization is shown by arrows. The results show that integrin α10β1 colocalizes with an increased expression of NeuN and GFAP on neurons and astrocytes respectively (regenerative response) but not with the microglial marker Iba1.

### Definitions

As used herein, the terms "rodent" and "rodents" refer to all members of the phylogenetic order *Rodentia.*

"Integrin alpha 10" or "Integrin alpha 10 subunit" as used herein refers to the alpha 10 subunit of the heterodimeric protein integrin alpha 10 beta 1. This denotation does not exclude the presence of the beta 1 subunit bound to the alpha 10 subunit thus forming the quaternary structure of integrin alpha 10 beta 1 heterodimer.

"Anti-integrin alpha 10 antibody" or "Anti-integrin alpha 10 subunit antibody" as used herein refers to an antibody capable of recognizing and binding to at least the alpha 10 subunit of the heterodimeric protein integrin alpha 10 beta 1. These antibodies may be antibodies that recognize an epitope of the heterodimeric protein integrin alpha10 beta1, wherein the epitope comprises amino acid residues of both the alpha10 and the beta1 subunit.

The term "identifying" as used herein refers to the action of recognizing a cell as being a certain type of cell, e.g. a neural stem cell or a neural progenitor cell. An alternative term to identifying is "detecting", which is used herein with the same meaning. A cell is identified as a neural stem cell or a neural progenitor cell for example by detecting expression of specific markers by the cell.

The terms "isolating", "sorting" and "selecting" as used herein refer to the action of identifying a cell as being a certain type of cell and separating it from cells that do not belong to the same cell type or to a differentiation state.

The term "scoring" as used herein refers to scoring of the integrin alpha10 subunit expression. The scoring may be measuring integrin alpha10 subunit expression via for example immunoassay, flow cytometry, immunofluorescence, western blot or immunoprecipitation in the sample population and comparing the measurement with a measurement done in a reference cell population expressing the integrin alpha10 subunit, as well as to a cell population not expressing the integrin alpha10 subunit. Examples of reference cells expressing the integrin alpha10 are C2C12 cells, HEK293 cells transfected with integrin sequences. Examples of reference cells not expressing alpha10 are non-transfected C2C12 cells and HEK293 cells.

The term "murine" refers to any and all members of the family *Muridae,* including rats and mice.

The term "substantially free from" is herein intended to mean below detection limits of the assay used thereby appearing negative, i.e. free from.

The term "committed" is herein intended to mean dedicated to, or focused on. Thus, a committed cell is a cell that is dedicated to, or focused on a specific differentiation pathway. From this it will follow that an uncommitted cell is not dedicated to, or focused on, any specific differentiation pathway and has several options.

The term "subject" used herein is taken to mean any mammal to which neural stem cells and/or neural progenitor cells and/or mesenchymal stem cell identified and/or isolated according to the methods disclosed herein, which are based on the detection of integrin alpha10 expression on the surface of the cells or intracellular in the cells, may be administered. Subjects specifically intended for treatment with the method of the disclosure include humans, as well as nonhuman primates, sheep, horses, cattle, goats, pigs, dogs, cats, rabbits, guinea pigs, hamsters, gerbils, rats and mice, as well as the organs, tumors, and cells derived or originating from these hosts.

### Detailed description

The invention is defined by the appended claims. Any embodiment not falling under the scope of the appended claims does not form part of the invention.

### Integrin alpha10 as a marker for neural stem cells and neural progenitor cells

The present inventors have surprisingly found that the integrin alpha10beta1 is present on human neural stem cells (NSCs) and/or neural progenitor cells (NPCs). Thus, this integrin can be used to identify, select and specifically isolate neural stem cells and neural progenitor cells from a mixed cell population and will be a useful tool in cell therapy to repair damaged tissue, for example as consequence of injuries of the nervous system or for treatment of neurodegenerative diseases. Compared to the above-mentioned NSC surface markers, integrin α10β1 identifies all three cell types of the adult mouse SVZ (see Table 1 above) suggesting that integrin α10β1 is a broader stem and progenitor cell marker, compared to other known markers, covering the different cellular subtypes of the brain stem cell niche.

The human integrin alpha10 chain sequence is known and publicly available at GenBank ^{™}/EBI Data Bank accession number AF074015. Thus, new uses and methods of the integrin alpha10 chain are disclosed in the present disclosure.

As revealed above, one aspect of the present disclosure relates to the use of a marker comprising an integrin alpha10 subunit expressed by a neural stem cell and/or a neural progenitor cell as a marker for mammalian neural stem cells and mammalian progenitor cells.

In one embodiment of the present disclosure, the integrin alpha10 subunit is expressed as a heterodimer in combination with an integrin beta1 chain.

In one embodiment of the present disclosure, the integrin alpha10 subunit is expressed on the cell surface of the mammalian NSC and/or NPC.

In one embodiment of the present disclosure, the integrin alpha10 subunit is expressed intracellularly in a mammalian NSC and/or NPC.

### A method for identifying neural stem cells (NSCs) and neural progenitor cells (NPCs)

One aspect of the present disclosure relates to an in vitro method for identifying a mammalian neural stem cell and/or a mammalian neural progenitor cell. The method comprises the steps of
a) detecting expression of an integrin alpha10 subunit by a cell comprised in a sample comprising neural tissue,
b) scoring the integrin alpha10 subunit expression of a), and
c) identifying the mammalian neural stem cell and/or the neural progenitor cell according to the scoring in b).

A further aspect of the present disclosure relates to an in vitro method for isolating a mammalian neural stem cell and/or a mammalian neural progenitor cell. The method comprises the steps of:
a) detecting expression of an integrin alpha10 subunit by a cell comprised in a sample comprising neural tissue,
b) scoring the integrin alpha10 subunit expression of a), and
c) selecting the mammalian neural stem cell and/or the mammalian neural progenitor cell according to the scoring in b).

In some embodiments of the present disclosure, the method of identifying a mammalian NSC and/or NPC and the method of isolating a mammalian NSC and/or NPC, further comprises a step of contacting the sample with an antibody which specifically binds integrin alpha10 subunit, prior to detecting integrin alpha10 subunit expression of b).

In more detail, the methods for identifying and/or isolating a mammalian neural stem cells and/or a mammalian neural progenitor cells according to the disclosure may further comprise the steps of:
e) providing a cell suspension comprising NSCs and/or NPCs,
f) contacting the cell suspension in e) with a monoclonal antibody or fragments thereof binding to the integrin alpha10beta1, under conditions wherein said monoclonal antibody or fragments thereof form an antibody-antigen complex with the extracellular domain of integrin alpha10beta1,
g) separating cells binding to said monoclonal antibody or fragments thereof in f), thereby producing a pure population of mammalian NSCs and/or NPCs

In some embodiments of the present disclosure, the methods for identifying and/or isolating a mammalian neural stem cells and/or a mammalian neural progenitor cells according to the disclosure may further comprise recovering the cells binding to the monoclonal antibody or fragments thereof from said antibody or fragments thereof.

The cell suspension provided in e) above, comprising mammalian NSCs and/or NPCs may be isolated from neural tissue as described in details in the section below "Neural tissue".

In some embodiments of the present disclosure, the methods for identifying and/or isolating a mammalian neural stem cells and/or a mammalian neural progenitor cells according to the disclosure is performed *in vitro.*

### Neural tissue

The main source of mammalian NSCs and NPCs is neural tissue. In fact, NSCs and NPCs are found in niches in fetal or adult mammalian central nervous system (CNS) and from fetal or adult mammalian brain or spinal cord where neurogenesis takes place.

The neural stem cell niche in brain is a tissue microenvironment capable of hosting and maintaining neural progenitor cells. Until recently, only two brain niches were recognized in mammals, the subventricular zone (SVZ) of the anterolateral ventricle and the subgranular zone (SGZ) of the hippocampal dentate gyrus. Increasing evidences show neurogenesis and gliogenesis also in other parts of the adult brain, particularly after injury, suggesting that additional stem cell niches are present in the adult brain (Lin and lacovitti, 2015). It was recently shown that leptomeninges host a subset of cells expressing markers of undifferentiated, proliferating and differentiating neural precursors presenting nestin and SOX2 and this set of cells persists in adulthood. Thus, meninges may represent another functional niche for progenitors during embryonic development and in adulthood.

Accordingly, in some embodiments of the present disclosure the neural tissue comprises NSCs and NPCs.

In some embodiments of the present disclosure, the neural tissue is derived from brain tissue.

In some embodiments of the present disclosure, the neural tissue is adult brain tissue.

In some embodiments of the present disclosure, the neural tissue is fetal brain tissue.

In some embodiments of the present disclosure the neural tissue is derived or obtained from the subventricular zone (SVZ), the subgranular zone (SGZ) or the meninges of a mammal.

All mammals are suitable for obtaining neural tissue. In some embodiments of the present disclosure, the neural tissue is selected from the group consisting of human, canine, equine, bovine, feline, murine, ovine or swine neural tissue. Other mammalian neural tissues may be obtained if there is a need for that.

In some embodiments of the present disclosure, the mammalian NSCs and NPCs are human NSCs and NPCs.

In one further embodiment of the present disclosure, the mammalian NSCs and NPCs are murine NSCs and NPCs.

### Detection of integrin alpha 10 expression

A key step in the method for identification of a mammalian neural stem cell and/or a mammalian neural progenitor cell, as well as for their isolation, is the detection of integrin alpha10 protein expression.

In one embodiment of the present disclosure, the detection of expression of an integrin alpha10 subunit by a cell is determined by flow cytometry. For example, the expression of alpha10 may be analyzed by flow cytometry, or any other methodology having high specificity. Multi-color analyses may be employed with the flow cytometry, which is particularly convenient. NSCs and/or NPCs may, thus, be separated from other cells on the basis of the level of staining for the particular antigens.

In one embodiment of the present disclosure, the detection of expression of an integrin alpha10 subunit by a cell is determined by measuring integrin alpha10 protein expression. For example, flow cytometry, immunofluorescence, immunoprecipitation and/or western blotting may be used.

In one embodiment of the present disclosure, the detection of expression of an integrin alpha10 subunit by a cell is determined by measuring integrin alpha10 mRNA expression. Detection of mRNA expression of a specific protein is well known to the skilled man in the art, and is generally done by probing the mRNA with a DNA or RNA probe specific for the mRNA of interest, under hybridization conditions where the probe is not hybridizing to other mRNA molecules. Different polymerase chain reactions (PCR) may also be used, which is obvious to the skilled man in the art.

A suitable PCR-method is given below. In brief, polymerase chain reaction (PCR) may be used.

RNA may be prepared from human neural stem cells or neural progenitor cells by standard methods, for example by the use of RNeasy Mini Kit (Qiagen Germany).

cDNA may be produced by reverse transcriptase reaction, Superscript II (Invitrogen, USA) according to manufacturer's recommendation with oligo d(T)-primers or gene specific primers.

PCR is thereafter performed to amplify the cDNA. Specific primers for α10, forward 5'GCT CCA GGA AGG CCC CAT TTG TG 3' and reverse 5'GTG TTT TCT TGA AGG GTG CCA TTT 3' are added to the cDNA and the specific product is amplified by Platinum Taq DNA polymerase (Invitrogen, USA) according to manufacturer's recommendations at 65° for 40 cycles.

Several methods are known to the person skilled in the art for detection of expression of markers. Accordingly, in one embodiments of the present disclosure the detection of expression of an integrin alpha10 subunit by a cell is determined by a method selected from the group consisting of immunoassay, flow cytometry, immunofluorescence, immunoprecipitation and western blot.

In still a further embodiment of the present disclosure, the integrin chain alpha10 expression is detected on the cell surface of a NSC and/or of a NPC or intracellular in a NSC and/or in a NPC in the method according to the disclosure. Methods given above, e.g. flow cytometry and immunoprecipitation may be used. Preferably flow cytometry is used.

In still a further embodiment of the present disclosure, the expression of the integrin alpha10 subunit is detected by any immunoassay, such as the methods described in Immunochemical protocols (Methods in molecular biology, Humana Press Inc). The detection may be performed by various methods, e.g. any immune method known to the skilled man in the art, such as immunoprecipitation, western blotting, magnetic-activated cell sorting (MACS) or flow cytometry methods, e.g. fluorescence activated cell sorting (FACS).

Accordingly, in some embodiments of the present disclosure, the expression of an integrin alpha10 subunit by a cell is detected via an antibody, wherein the antibody is a monoclonal antibody, polyclonal antibody, a chimeric antibody, a single chain antibody or fragment thereof.

Antibodies, such as monoclonal antibodies or fragments thereof, are particularly useful for identifying markers, cell surface proteins as well as intracellular markers, associated with particular cell lineages and/or stages of differentiation. Thus, it is suitable for the identification of integrin alpha10.

In one embodiment of the present disclosure, the antibody is a monoclonal antibody or a polyclonal antibody.

In a further embodiment of the present disclosure, the antibody is a non-human antibody, a chimeric antibody, a bispecific antibody, a humanized antibody or a human antibody.

Still, identification may as well be performed by any specific molecule, such as a protein or peptide, binding specifically to the integrin alpha10 molecule. Examples of such proteins or peptides are natural ligands, binding to the integrin alpha10 molecule. Such natural ligands may be made recombinant, chemically synthesized, or purified from a natural source.

The detection is facilitated when the antibody, protein or peptide, binding specifically to the integrin alpha10 molecule is bound to a detectable moiety.

In some embodiments of the present disclosure, an antibody is used for detection of the expression of integrin alpha10 subunit and the antibody is covalently bound to a detectable moiety, such as a detectable moiety selected from the group consisting of a fluorophore, an enzyme or a radioactive tracer or radioisotope.

In some embodiments of the present disclosure, the antibody is bound to a fluorophore and the fluorophore is selected from the group consisting of fluorescein isothiocyanate, phycoerythrin and phycoerythrin conjugates, allophycocyanin and allophycocyanin conjugates, Texas Red and Texas Red conjugates, Alexa series of fluorochromes, Brilliant Violet and Brilliant Blue series of fluorochromes.

Many other fluorophores may be used and the skilled person will choose the most suitable one according to the specific detection method and also according to the characteristics of the antibody used.

In further embodiments of the present disclosure, the antibody has an isotype selected from the group consisting of IgA, IgD, IgG and IgM.

In even further embodiments of the present disclosure, the antibody is:
a) a monoclonal antibody, produced by the hybridoma cell line deposited at the Deutsche Sammlung von Mikroorganismen und Zellkulturen GmbH under the accession number DSM ACC2583; or
b) an antibody which competes for binding to the same epitope as the epitope bound by the monoclonal antibody produced by the hybridoma deposited at the Deutsche Sammlung von Mikroorganismen und Zellkulturen GmbH under the accession number DSM ACC2583; or
c) a fragment of a) or b), wherein said fragment is capable of binding specifically to the extracellular I-domain of the integrin alpha 10 subunit chain.

The detection may also be facilitated when the antibody, protein or peptide, binding specifically to the integrin alpha10 molecule is bound to a solid support. Accordingly, in one embodiment of the present disclosure an antibody is used for detection of the expression of integrin alpha10 subunit and the antibody is attached to a solid support.

### Isolation and cultivation of NSCs and NPCs

The isolation of a mammalian neural stem cell (NSC) and/or a mammalian neural progenitor cell (NPC) according to the method of the present disclosure may be based on the cells capacity to adhere to plastic culture dishes and form colonies under specific culture conditions. Suitable protocol for isolation of mammalian neural stem cells and neural progenitor cells, without including the marker according to the disclosure, is further given in detail in Giachino et al. 2009, Guo W et al., (2012) and Oliver-De la Cruz and Ayuso-Sacido (2012). Thus, known methods may be used, but with the introduction of the marker according to the disclosure.

Procedures for separation may include magnetic separation, using e.g. antibody-coated magnetic beads, affinity chromatography, agents joined to a monoclonal antibody or used in conjunction with a monoclonal antibody, e.g., complement and cytotoxins, and "panning" with antibody attached to a solid matrix,
e.g., a plate, or other convenient techniques. Techniques providing accurate separation include fluorescence activated cell sorters, which can have varying degrees of sophistication, e.g., a plurality of color channels, light scattering detecting channels, impedance channels, etc. known to the skilled man in the art.

Further protocols for separation methods suitable to be used in the method according to the disclosure are described by Orfao, A and Ruiz-Arguelles, A ((1996) General Concepts about Cell Sorting Techniques. Clin Biochem. 29(1):5-9), and by Herzenberg, LA, De Rose, SC and Herzenberg, LA ((2000) Monoclonal Antibodies and FACS: complementary tools for immunobiology and medicine. Immunol. Today. 21(8):383-390).

Mammalian NSCs and NPCs are first purified from the mixture of cells collected from nervous tissue. Generally, negative markers are used to separate NSCs and NPCs from differentiated cells.

The isolation of NSCs and NPCs from other cells, for example committed neural cells, present in neural tissue comprises a selection and sorting step where the NSCs and NPCs are first identified and then separated from the other cells. Various techniques known to the skilled artisan may be employed to separate the cells by initially removing cells dedicated to other lineages than NSCs and NPCs

In a first separation, antibodies for other markers may be used labelled with one or more fluorochrome(s).

The NSCs and the NPCs may be selected against dead cells, by employing dyes associated with dead cells (propidium iodide, LDS). The cells may be collected in their culture medium, or in any physiological saline solution, preferably buffered, such as phosphate buffer saline (PBS), optionally with fetal calf serum (1% FCS) or bovine serum albumin (1% BSA) present

Markers that are not expressed on NSCs and NPCs are, e.g. CD326, CD34 and CD45 and their expression, or lack of expression, may in certain embodiments of the present disclosure be used for as markers for negative selection of cells that are not NSCs or NPCs.

If further lineages or cell populations not being NSCs or NPCs are to be removed in one step, various antibodies to such lineage-specific markers may be included.

In some embodiments of the present disclosure, other less specific and non-unique mammalian NSCs and/or NPCs markers may be analyzed in parallel with the marker according to the disclosure. In fact, NSCs and NPCs subsets detected at different stages of CNS development have been shown to express markers such as nestin, GFAP, CD15, CD24, Sox2, Musashi, CD133, EGFR, PDGFRα, Doublecortin (DCX), Pax6, FABP7 and GLAST. These markers are co-expressed by some of the cells expressing integrin alpha10 subunit, as shown in the examples. However, none of these markers are uniquely expressed by NSCs and/or by NPCs, many are indeed expressed by neural differentiated cells and other non-neural cell types.

In contrast, the use of the integrin alpha10 marker according to the disclosure in the isolation and expansion protocols will give a homogenous population of neural stem cells and/or neural progenitor cells with the ability to differentiate to different cells of the brain and spinal cord thus being useful for regenerating brain or spinal cord tissue.

Thus, including the marker according to the disclosure, comprising an integrin alpha10 subunit in known isolation and expansion protocols, as well as using the marker(s) alone, will be highly valuable for further evaluation and enrichment of the neural stem cells and neural progenitor cell populations. Particularly, no other specific and unique marker as the marker according to the disclosure for mammalian neural stem cells and neural progenitor cells is known.

NSCs and NPCs may as well be selected based on light-scatter properties and their expression of various cell surface antigens, in combination with the identification using the method according to the disclosure.

Generally, markers present on the cell surface or intracellularly are detected with the help of fluorochromes. Fluorochromes, which may find use in a multi-color analysis, include phycobiliproteins, e.g., phycoerythrin and allophycocyanins, fluorescein, Texas red, and many others, all well known to the skilled man in the art and commercially available.

Commonly used techniques for detection and selection of cells based on the use of markers present on their cell surface are flow cytometry and immunoprecipitation.

In some embodiments of the present disclosure, NSCs and NPCs may be analyzed by flow cytometry or immunoprecipitation thereby detecting and identifying integrin alpha10 subunit expression. The person skilled in the art is familiar with suitable protocols for flow cytometry and immunoprecipitation to be used for detection and/or selection of cells.

If a population of cells is collected from whole mouse brain, only a minor fraction, for example less than 2% of the total number of cells are NSCs or NPCs.

If an antibody or fragments thereof is used it may be attached to a solid support to allow for a highly specific separation. The particular procedure for separation employed, e.g. centrifugation, mechanical separation, such as columns, membranes or magnetic separation, should maximize the viability of the fraction to be collected. Various techniques of different efficacy may be employed known to a person skilled in the art. The particular technique employed will depend upon efficiency of separation, cytotoxicity of the methodology, ease and speed of performance, and necessity for sophisticated equipment and/or technical skill.

Procedures for separation of NSCs and/or NPCs from a cell suspension aided by the method according to the disclosure may include magnetic separation, using e.g. antibody-coated magnetic beads, affinity chromatography based on the antibody or fragments thereof according to the disclosure, and "panning" with an antibody or fragments thereof attached to a solid matrix, e.g., a plate, or other convenient techniques.

Techniques for providing accurate separation include fluorescence activated cell sorters, magnetic bead sorting and any suitable method known by those of skill in the art.

In a further embodiment of the present disclosure, the first enrichment step of the methods of the present disclosure, is a positive selection of the NSCs or of the NPCs that may be repeated until the desired purity of the NSCs or of the NPCs is achieved.

### A method for producing an isolated population of cells enriched for mammalian NSCs and/or NPCs

One aspect of the present disclosure relates to a method for manufacturing an isolated population of mammalian cells in vitro which are enriched for neural stem cells and/or neural progenitor cells relative to a reference population, the method comprising the steps of
a) providing at least a portion of a population of cells, or a portion of a reference population, comprising a neural stem cell and/or a neural progenitor cell,
b) introducing into the population of cells in a) above a compound identifying an integrin alpha10 subunit expressed by the neural stem cell and/or neural progenitor cell,
c) selecting and isolating from the population of cells in b) above the neural stem cells and/or the neural progenitor cells,
thereby producing a population of cells enriched for neural stem cells and/or neural progenitor cells.

Providing a population may be performed in a similar way as in the method for identification of NSCs and NPCs described in detailed above. The population of cells may comprise at least one NSC and/or at least one NPC, or may comprise only cells that are neither NSCs nor NPC. For example a population of cells obtained or derived from neural tissue may be provided, or a reference population of cells, such as HEK cells or C2C12 cells transfected with integrin alpha10 may be provided.

The compound introduced to identify the NSCs and/or the NPCs may be a protein, peptide, monoclonal antibody, or part thereof, or polyclonal antibody identifying the NSCs and/or NPCs.

The compound introduced to identify the NSCs and/or the NPCs may be a protein, peptide, monoclonal antibody, or part thereof, or polyclonal antibody that is able to detect integrin alpha10.

In one embodiment of the present disclosure, the NSCs and/or NPCs is identified as a NSC and/or as a NPC by detecting expression of integrin chain alpha10 on the cell surface of said NSC and/or of said NPC according to the method for identifying NSCs and/or NPCs described above.

Monoclonal or polyclonal antibodies, or parts thereof, are particularly useful for identifying markers, e.g. markers expressed on the cell surface of intact viable cells. The compound used to identify the NSC and/or NPC may also be used for the separation step. Thus, said compound(s), such as antibodies, or parts thereof, may be attached to a solid support to allow for a first crude separation. Examples of solid supports are beads e.g. magnetic beads, agarose or other similar types of beads known to the skilled man in the art. Any means suitable for separation of cells may be employed on the condition that the separation is not unduly detrimental to the viability of a cell.

The separation techniques employed should maximize the retention of viability of the fraction to be collected. The assessment of viability is described below.

In brief, assessment of cell viability may be performed using e.g. flow cytometry. After staining of the appropriate cell viability dye can be added to discriminate between viable and non-viable cells. A number of such dyes exist, of which examples and typical methods for using them are described. The principle is the same for most of these dyes: these dyes enter the cells if the cell membrane is compromised; as such, cells that stain with these dyes are non-viable, and cells that do not stain are considered viable.

Examples of dyes are Propidium Iodide (PI), 7-Aminoactinomycin D (7 AAD), To-Pro3, and Ethidium Monoazide (EMA). Other methods are described in Flow Cytometry and Cell Sorting (Springer Lab Manual) by A. Radbruch (Springer Verlag, 2nd edition, January 2000).

The cell viability of the fraction collected is typically > 90%, preferably 95%, 96%, 97%, 98%, 99%, 99.9%, or even 100%.

The particular technique employed for separation of cells in the method according to the disclosure will depend upon efficiency of separation, cytotoxicity of the methodology, ease and speed of performance, and necessity for sophisticated equipment and/or technical skill.

In one embodiment of the method according to the present disclosure, at least one enrichment step of mammalian NSCs and/or NPCs is included.

In one embodiment of the method according to the present disclosure, at least one enrichment step of mammalian NSCs is included.

In one embodiment of the method according to the present disclosure, at least one enrichment step of mammalian NPCs is included.

In still a further embodiment of the present disclosure, the first enrichment step of NSCs and/or NPCs is a negative selection of the NSCs and/or NPCs, i.e. other lineage-committed cells are depleted, or removed, from the initial population of cells. For example, cells expressing markers such as CD34, CD45 and CD326 are negatively selected.

In still a further embodiment of the present disclosure, the methods of the present disclosure comprise a further step a negative selection based on detection of the expression of integrin alpha11 subunit, for example, cells expressing the marker integrin alpha11 are depleted, or removed, from the population of cells.

In still a further embodiment of the present disclosure, the first enrichment is a positive selection of NSC and/or NPCs that may be repeated till the desired purity of the NSC and/or of the NPC is achieved. For a positive or a negative selection, proteins, peptides, monoclonal or polyclonal antibodies may be used as a compound to identify the integrin alpha10 molecule as described above. The compound may be conjugated with means for separation, such as magnetic beads, which allow for direct separation; biotin, which can be removed with avidin; or streptavidin bound to a support; fluorochromes, which can be used with a fluorescence activated cell sorter; or the like, to allow for ease of separation of the particular cell type as exemplified in the paragraphs above. Any technique may be employed which is not unduly detrimental to the viability of the cells of interest, i.e. the NSCs and the NPCs.

In one embodiment of the present disclosure, the selection is performed by fluorescent cell sorting, by using e.g. a flow cytometry based cell sorter such as a FACS^{®}, or any other similar methodology having high specificity. Multi-color analyses may be employed with flow cytometry which is particularly convenient and the technique well known to person skilled in the art of flow cytometry. The cells may be separated on the basis of the level of staining for the particular antigens. In a first separation, antibodies for other markers may be used labelled with one fluorochrome, while the antibodies for the dedicated lineages, i.e. the integrin alpha10, may be conjugated to (a) different fluorochrome(s). Other markers may in further embodiments of the present disclosure be nestin, GFAP, CD15, CD24, Sox2, Musashi, CD133, EGFR, PDGFRα, Doublecortin (DCX), Pax6, FABP7 and GLAST that NSC and/or NPC may express. Examples of markers that are not expressed on NSC and NPC are CD326, CD34, CD45 and integrin alpha11 and their expression, or lack of, may in further embodiments of the present disclosure also be evaluated together with the marker according to the disclosure, e.g. integrin alpha10 expression.

If further lineages or cell populations are to be removed in this step, various antibodies to such lineage specific markers may be included. Fluorochromes which may find use in a multi-color analysis include phycobiliproteins, e.g., phycoerythrin and allophycocyanins, fluorescein, Texas red, and any suitable fluorochrome known by those of skill in the art.

The cells may be selected against dead cells, by employing dyes associated with dead cells such as propidium iodide or LDS-75 1 (Laser Dye Styryl-75 1 (6-dimethylamino-2-14-[4-(dimethylamino)phenyl]-1 ,3-butadienyll-1-ethyl quinoliniurn perchlorate) ). The cells may be collected in any suitable cell culturing media, such as Iscove's modified Dulbecco's medium (IMDM), or in any physiological saline solution, preferably buffered, such as phosphate buffer saline (PBS), optionally with fetal calf serum (FCS) or bovine serum albumin (BSA) present. Other techniques for positive or negative selection may be employed, which permit accurate separation, such as affinity columns, and the like, further described by Silvestri F, Wunder E, Sovalat H, Henon P, Serke S in Positive selection of CD34+ cells: a short review of the immunoadsorption methods currently available for experimental and clinical use (Report on the "2nd European Workshop on stem Cell Methodology", Mulhouse, France, May 3-7, 1993. J Hematother. 1993 Winter;2(4):473-81) and by Basch RS, Berman JW, Lakow E.in Cell separation using positive immunoselective techniques (J Immunol Methods. 1983 Feb 11;56(3):269-80).

Cells may be selected based on light-scatter properties as well as their expression of various cell surface antigens.

While it is believed that the particular order of separation is not critical to this
disclosure, the order indicated is one way of performing the disclosure that is known to work. Thus, suggestively, cells are initially separated by a crude separation, preferably a negative selection removing cells not committed for NSCs and NPCs using negative cell markers such as CD326, CD34 and CD45. The negative selection is typically followed by a positive selection, wherein the positive selection is of a marker associated with NSC and/or the NPCs and negative selection for markers associated with lineage committed cells, and other stem cell populations not being NSC and/or the NPCs. This separation is then followed by selection for a cellular population, or a cellular composition comprising said population, having multi-lineage potential as a NSC and/or a NPC and enhanced self-regeneration capability. The composition is further described below.

In further embodiments of the present disclosure, the enrichment of such a population is about 70, 80, 90, 95, 98, 99, 99.9, or even 100%. The viability of such cells is discussed in detail above.

The enriched population may further be expanded and then induced with defined factors, such as epidermal growth factor (EGF) and fibroblast growth factor-2 (FGF-2), to differentiate into specific neural cells. For example, FGF-2 is generally used in combination with heparin, which mediates the binding of the growth factor to its receptor. Other growth factors that have been reported to support cell culture are Transforming growth factor alpha (TGF-α), Leukemia inhibitory factor (LIF) and its equivalent Ciliary neurotropic factor (CNTF), or Brain-derived neurotrophic factor (BDNF). PDGFα is frequently used in the maintenance media for oligodendrocyte progenitor cells. As alternative or in addition to using specific growth factors, NPCs and NSCs may be co-cultured in presence of other supportive cells like astrocytes, that seem to favor the NPCs and NSCs growth by physical contact, or endothelial cells, that may enhance cell proliferation via vascular endothelial growth factor (VEGF) production.

### Modulation of NSC and/or NPC

A further aspect of the present disclosure relates to a method for determining whether a test compound modulates a mammalian NSC and/or NPC differentiation *in vitro.* Such a method comprises the steps of
a) providing a neural stem cell and/or a neural progenitor cell that expresses integrin alpha10 subunit,
b) contacting the neural stem cell and/or the neural progenitor cell with a test compound, and
c) detecting a change in rate or pattern of differentiation of the neural stem cell and/or neural progenitor cell as an indication of that the test compound modulates a neural stem cell and/or a neural progenitor cell differentiation,
wherein the rate or pattern of differentiation is determined by detecting integrin alpha10 expression by the cell according to the method disclosed herein, see also the section above "Detection of integrin alpha10 expression".

The NSCs and the NPCs provided may be an enriched cell population achieved according to any of the methods disclosed herein, the isolated NSC and/or NPC according to the disclosure, or the cellular composition according to the disclosure.

The test compound may be any compound known to affect or suspected to affect NSC and/or NPC, e.g. pharmaceutical compositions, drugs, polyclonal or monoclonal antibodies, or parts thereof, such as antibodies binding to integrin alpha10 or any other molecule on the NSC and/or on the NPC, factors used to promote growth of NSC and/or of NPC, e.g. PDGFRα, EGF and FGF-2.

The detection of a change in rate or pattern of e.g. differentiation of the NSC and/or of the NPC as an indication that the test compound modulates NSC and/or NPC differentiation may be done by detecting integrin alpha10 expression on the cell surface of said neural stem cell and/or a neural progenitor cell or intracellular in a neural stem cell and/or a neural progenitor cell according to the methods disclosed herein.

The detection of a change in rate or pattern of e.g. differentiation of the NSC and/or of the NPC as an indication that the test compound modulates NSC and/or NPC differentiation may be done via flow cytometry or any other suitable method, such as any immuno-method, known to a person skilled in the art. The change in rate or pattern of differentiation may be detected via kinetic, functional or phenotypical studies of the NSC and/or NPC modulated with the test compound, relative to an untreated, or mock treated, NSCs and/or NPCs population.

### A cellular composition

One aspect of the present disclosure relates to an *in vitro* cell culture of undifferentiated mammalian cells expressing an integrin alpha10 subunit, wherein the cells are derived from neural tissue and wherein
a) cells in the culture have the capacity to differentiate into neurons and/or oligodendrocytes and/or astrocytes when differentiated in a culture medium substantially free of both serum and a proliferation-inducing growth factor as defined in (b) to produce a cell culture of at least 10% neurons and/or oligodendrocytes and/or astrocytes;
b) the cell culture divides in a culture medium containing a serum replacement such as B27 and at least one proliferation-inducing growth factor;
c) cells in the culture differentiate into neurons and/or oligodendrocytes and/or astrocytes upon withdrawal of both serum replacement and the proliferation inducing growth factors.

In one embodiment of the present disclosure, step c) comprises addition of fetal calf serum.

A further aspect of the present disclosure relates to an *in vitro* cell culture comprising
a) a culture medium containing a serum replacement such as B27 and at least one proliferation-inducing growth factor; and
b) undifferentiated mammalian cells derived from the central nervous system of a mammal, wherein at least 10%, preferably at least 20%, preferably at least 30%, preferably at least 40%, preferably at least 50%, preferably at least 60%, preferably at least 70%, preferably at least 80%, preferably at least 90% of the cells express an integrin alpha10 subunit.

An even further aspect of the present disclosure relates to a suspension culture of mammalian undifferentiated cells expressing an integrin alpha10 subunit, wherein said cells are substantially formed into cell aggregates, and wherein the cell aggregates are maintained in a culture medium containing a proliferation-inducing growth factor.

In some embodiments of the present disclosure, integrin alpha10 expression is as described in the section above "Integrin alpha10 as a marker for neural stem cells and neural progenitor cells".

In some embodiments of the present disclosure, at least 10%, preferably at least 20%, preferably at least 30%, preferably at least 40%, preferably at least 50%, preferably at least 60%, preferably at least 70%, preferably at least 80%, preferably at least 90% of the undifferentiated mammalian cells are mammalian neural stem cells expressing an integrin alpha10 subunit.

In some embodiments of the present disclosure, the undifferentiated mammalian cell is a neural stem cell or a neural progenitor cell.

In some embodiments of the present disclosure, the undifferentiated mammalian cell is obtained or derived from adult or fetal mammalian, for example human or murine, neural tissue, which is described in detail in the section above "Neural tissue".

In some embodiments of the present disclosure, some of the undifferentiated mammalian cells further express at least one marker selected from the group consisting of nestin, PSA-NCAM, GFAP, SOX2 and PDGFRα.

Compositions having greater than 50%, such as greater than 60%, e.g. greater than 70%, such as greater than 80%, e.g. greater than 90%, such as greater than 95%, such as 96%, 97%, 98%, or 99.9%, of human NSC or NPC cells may be achieved according to the disclosed methods for enrichment of NSC or NPC. Such NSC and/or NPC are able to provide for cell regeneration and development of members of all of the various lineages of NSC, such as neurons, astrocytes, and oligodendrocytes and other cells.

Ultimately, a single cell type may be obtained from a NSC or a NPC composition and used for reconstitution or regeneration of a mammalian neural tissue. The NSC or NPC composition should preferably be administered in a therapeutically effective dosage, wherein the dosage is a specific cell number able to repopulate said mammal, such as a human being. The cell number may be different from donor to donor and may be determined empirically from case to case by a person skilled in the art.

Cell proliferation-inducing factors are specific for each cell type and are known to the person of skill in the art. In some embodiments of the present disclosure, the at least one proliferation-inducing growth factor is selected from the group consisting of epidermal growth factor (EGF), fibroblast growth factor-2 (FGF-2), Transforming growth factor alpha (TGF-α), Leukemia inhibitory factor (LIF), Ciliary neurotropic factor (CNTF), Brain-derived neurotrophic factor (BDNF), PDGFα, and combinations thereof.

### Treatment of injuries of the nervous system and neurodegenerative diseases

Mammalian NSC and/or NPC, such as human or mouse NSC and/or NPC identified and isolated according to the methods disclosed herein can be used for treating damage and injuries and/or preventing and protecting from damage of the nervous system and/or treating neurodegenerative disease in a subject in need thereof. As for neurodegenerative disorders, this applies in particular to neurodegenerative diseases which are associated with loss or damage of neuronal tissue. The present disclosure treats such neurodegenerative diseases by regenerating the lost or damaged tissue.

Furthermore, mesenchymal stem cells (MSCs) identified and isolated according to methods disclosed e.g. in WO 03/106492, can also be used for regenerating lost or damaged tissue of the central nervous system as evidenced by Steinberg G.K. et al (2016) Stroke 47:1817-1824. Thus, in one aspect the present disclosure relates to a methods of regenerating lost or damaged tissue of the central nervous system, and to methods of treating disease or damage and/or preventing and protecting from damage of the central nervous system in a subject in need thereof, the method comprising:
a) providing a composition comprising an enriched population of mammalian neural stem cells and/or mammalian neural progenitor cells, wherein the cells express an integrin alpha10 subunit;
b) administering a therapeutically effective amount of the isolated population of mammalian neural stem cells and/or neural progenitor cells to the subject, thereby treating the disease or damage and/or preventing and protecting from damage of the central nervous system.

A further aspect of the present disclosure relates to a method of treating a mental and behavioral disorder in a subject in need thereof, the method comprising:
a) providing a composition comprising an enriched population of mammalian neural stem cells and/or mammalian neural progenitor cells, wherein the cells express an integrin alpha10 subunit;
b) administering a therapeutically effective amount of the isolated population of mammalian neural stem cells and/or neural progenitor cells to the subject, thereby treating the neurologic disorders with psychiatric symptoms.

A further aspect of the present disclosure relates to a method of treating disease or damage and/or preventing and protecting from damage of the nervous system in a subject in need thereof, the method comprising:
a) providing a composition comprising an enriched population of mammalian mesenchymal stem cells, wherein the cells express integrin alpha10 subunit;
b) administering a therapeutically effective amount of the isolated population of mammalian mesenchymal stem cells to the subject,
thereby treating the disease or damage and/or preventing and protecting from damage of the central nervous system.

A further aspect of the present disclosure relates to a method of treating a mental and behavioral disorder in a subject in need thereof, the method comprising:
a) providing a composition comprising an enriched population of mammalian mesenchymal stem cells, wherein the cells express integrin alpha10 subunit;
b) administering a therapeutically effective amount of the isolated population of mammalian mesenchymal stem cells to the subject,
thereby treating the neurologic disorders with psychiatric symptoms.

Said mesenchymal stem cells may be isolated e.g. from bone marrow, adipose tissue, cord blood, Wharton's jelly, dental pulp, amniotic fluid, amniotic membrane, dental tissues, endometrium, limb bud, blood, placenta and fetal membrane, salivary gland, skin and foreskin, sub-amniotic umbilical cord lining membrane or synovial membrane.

A further aspect of the present disclosure relates to a marker for mammalian neural stem cells and/or mammalian neural progenitor cells, comprising an integrin alpha10 chain subunit expressed by the neural stem cell and/or neural progenitor cells, for use in a method of treating a disease or damage and/or preventing and protecting from damage of the nervous system and/or of treating a mental and behavioural disorder.

A further aspect of the present disclosure relates to a composition comprising an isolated population of mammalian neural stem cells and/or mammalian neural progenitor cells expressing an integrin alpha10 subunit for use in a method of treatment of disease or damage of the nervous system and/or mental and behavioral disorder.

In some embodiments of the present disclosure, integrin alpha10 expression is as described in the section above "Integrin alpha10 as a marker for neural stem cells and neural progenitor cells".

In some embodiments of the present disclosure, the population of cells is enriched for mammalian neural stem cells and/or mammalian neural progenitor cells expressing an integrin alpha10 subunit, for example as described in the section above "A cellular composition" and "A method for producing an isolated population of cells enriched for mammalian NSCs and/or NPCs".

Neural stem cells, such as a neural progenitor cell, such as mesenchymal stem cells isolated by detecting integrin alpha10 subunit expression on the cell surface of said cells according to the methods disclosed herein may be implanted in a damaged area of the nervous system of a subject and may so promote growth of neural tissues and repair injuries of the nervous system and neurodegenerative diseases.

The neural stem cells, such as the neural progenitor cell, such as the mesenchymal stem cells isolated by detecting integrin alpha10 subunit expression on the cell surface of said cells according to the methods disclosed herein and implanted or transplanted to a subject in need thereof may promote neural stem cell migration and differentiation and production of extracellular matrix factors that provide trophic support for damaged cells.

In some embodiments of the present disclosure, the neural stem cells, such as the neural progenitor cell, such as the mesenchymal stem cells isolated by detecting integrin alpha10 subunit expression on the cell surface of said cells according to the methods disclosed herein are administered to a subject in need thereof via intracerebral, intra-arterial, intravenous, or intracerebroventricular routes.

The cells may be administered to said subject in one or more occasions.

Once they have been administered or transplanted into a subject, the cells will behave differently according to the environment they are transferred to and may differentiate into all type of neural cells.

### Diseases, damages and disorders to be characterized and treated

Several diseases and disorders can be characterized and treated using the markers and/or cells and methods of the present disclosure.

In one embodiments of the present disclosure, the disease or damage of the nervous system is an injury of the central or peripheral nervous system or a neurodegenerative disease. Said disease, damage or injury may involve lost or damaged neural cells.

In some embodiments of the present disclosure, the injury of the nervous system involves injury to the brain, brain stem, the spinal cord, and/or peripheral nerves, resulting in conditions such as stroke, traumatic brain injury (TBI), spinal cord injury (SCI), diffuse axonal injury (DAI), epilepsy, neuropathy, peripheral neuropathy, and associated pain and other symptoms that these syndromes may cause.

Stroke is a medical condition in which poor blood flow to the brain results in cell death. There are two main types of stroke: ischemic and hemorrhagic. Brain ischemia (a.k.a. cerebral ischemia, cerebrovascular ischemia) is a condition in which there is insufficient blood flow to the brain to meet metabolic demand. This leads to poor oxygen supply or cerebral hypoxia and thus to the death of brain tissue or cerebral infarction / ischemic stroke. Focal brain ischemia reduces blood flow to a specific brain region, increasing the risk of cell death to that particular area, whereas global brain ischemia affects the brain globally. Rodent models of focal cerebral ischaemia is frequently employed in experimental stroke research.

As described in (Fairbairn et al, 2015), NSCs can be implanted into peripheral nerve injury sites in consequences of an acute peripheral nerve injury. NSCs can also be implanted into chronically denervated nerve to improve morphological and electrophysiological recovery.

The present inventors have demonstrated (e.g. figures 13-14) that neural stem cells expressing integrin alpha 10 beta 1 are recruited in areas afflicted by stroke. In one embodiment, the present disclosure is thus useful for characterizing size and location of an area afflicted by ischemic damage such as stroke. The disclosure is thus useful for the medical professional, as a diagnostic and prognostic tool subsequent to disease or damage to the CNS. See e.g. Panagiotou et al (2015) Frontiers in Neuroscience Vol. 9, Article 182 which demonstrates use of nanoparticle-conjugated antibodies for assessing characterization of stroke. Furthermore, neural stem cells (e.g. autologous neural stem cells or progenitor cells) can be obtained from a donor, such as the patient himself, and characterized as neural stem or progenitor cells, expanded and transplanted into the damaged or diseased area of the CNS. Thus, the disclosure is useful for treating a multitude of diseases and disorders of the CNS, as well as injuries and trauma involving loss or damage of neural tissue.

In other embodiments of the present disclosure, the disease or damage of the nervous system is a neurodegenerative disease that involves the degeneration of neurons and their processes in the brain, brain stem, the spinal cord, and/or peripheral nerves, such as neurodegenerative disorders including but not limited to Parkinson's Disease, Alzheimer's Disease, senile dementia, Huntington's Disease, amyotrophic lateral sclerosis (ALS), neuronal/axonal injury associated with Multiple Sclerosis (MS), and associated symptoms.

In other embodiments of the present disclosure, the injury of the nervous system and/or the neurodegenerative disease involves dysfunction, and/or loss of neurons in the brain, brain stem, the spinal cord, and/or peripheral nerves, such as dysfunction and/or loss caused by metabolic diseases, nutritional deficiency, toxic injury, malignancy, and/or genetic or idiopathic conditions, including but not limited to diabetes, renal dysfunction, alcoholism, chemotherapy, chemical agents, drug abuse, vitamin deficiencies, infection, and associated symptoms.

In other embodiment of the present disclosure, the injury of the nervous system and/or the neurodegenerative disease involves the degeneration or sclerosis of glia such as oligodendrocytes, astrocytes, and Schwann cells in the brain, brain stem, the spinal cord, and peripheral nervous system, including but not limited to Multiple Sclerosis (MS), optic neuritis, cerebral sclerosis, post-infectious encephalomyelitis, and epilepsy, and associated symptoms.

In other embodiments of the present disclosure, the injury of the nervous system and/or the neurodegenerative disease involves the retina, photoreceptors, and associated nerves including but not limited to retinitis pigmentosa, macular degeneration, glaucoma, and associated symptoms.

In other embodiment of the present disclosure, the injury of the nervous system and/or the neurodegenerative disease involves the sensory epithelium and associated ganglia of the vestibuloacoustic complex, including but not limited to noise induced hearing loss, deafness, tinnitus, otitis, labyrintitis, hereditary and cochleovestibular atrophies, Meniere's Disease, and associated symptoms.

In some embodiments of the present disclosure the injury of the nervous system is selected from a group consisting of spinal cord injuries (SCI), traumatic brain injuries (TBI), stroke and brain cancer. In a preferred embodiment of the present disclosure, the injury of the nervous system is stroke.

In some embodiments of the present disclosure mental and behavioral disorders involving damage of neural tissue, can be treated by the NCSs or NPCs of the present disclosure. See e.g.

Ladran et al (2013) Interdiscip Rev Syst Biol Med. 5(6): 701-715 and Kalman et al (2016) Stem Cells Int. 7909176.

In certain embodiments of the present disclosure the mental and behavioral disorders are selected from the group consisting of Rett syndrome, schizophrenia, depression, autism spectrum disorders (ASD) and bipolar disorder (BPD). In fact, some mental and behavioral disorders are associated with neural cells having altered morphology and/or function. Therefore, transplantation of healthy NSCs and/or NPCs can result in the generation of morphologically and functionally healthy committed neural cells and in a regression of the mental and behavioral disorder.

The cellular composition according to the disclosure may also be used for treatment of genetic diseases. Genetic diseases associated with NSC and/or NPCs may be treated by genetic modification of autologous or allogeneic NSC to correct the genetic defect. For example, Huntington's disease (HD) is a hereditary disease and children with an affected parent have a 50% chance of inheriting the genetic fault that causes the disease. This fault occurs in the gene that holds the code for a protein called Huntingtin. The defective gene causes the body to make a faulty, toxic version of the Huntingtin protein and this eventually results in the loss of Medium spiny neuron (MSNs) and other neurons. Administration of a cellular composition comprising healthy NSCs and/or NPSc to a subject whose cells comprise the defective gene may result in the subject being able to produce healthy Huntingtin.

With allogeneic NSCs and/or NPCs, normal cells form a mammal of the same species without the genetic defect can be used as a therapy.

Various procedures can be contemplated for transferring and immobilizing the NSCs and/or the NPCs and/or the MSCs, and the composition comprising NSCs and/or NPCs and/or the MSCs, including injecting the isolated cells into the site of defect e.g. damage to brain or bone marrow, incubating isolated cells in suitable gel and implanting, incubating with bioresorbable scaffold, or by systemically infusing etc. Different procedures are known by the person skilled in the art.

Optionally NSCs and/or NPCs and/or the MSCs can be incubated with an antibody to the integrin alpha10 in order to hold the cells in place. Thus antibodies can be conjugated to a bioresorbable scaffold allowing immobilization of the cells before implantation into the damaged or defect site, e.g. into the site of a neural defect. The scaffold allows 3D immobilization of NSCs and/or NPCs and/or the MSCs. Suitable biomaterial scaffolds are exemplified below. The examples given are not limiting the use of other suitable scaffolds obvious to a skilled artisan to choose if more suitable for the particular application.

Types of scaffold include, bioresorbable poly(α-hydroxy esters) scaffolds such as polylactic acid (PLLA), polyglycolic acid (PGA) and copolymer (PLGA).

Further embodiments of the present disclosure include scaffolds derived from polymeric gels such as hyaluronic acid, collagen, alginate and chitosan.

### Examples

Examples not falling under the scope of the appended claims do not form part of the invention.

### Example 1: Isolation of cells from mouse subventricular zone (SVZ) and flow cytometry analysis

### SVZ isolation

Neural stem/progenitor cells were isolated from the SVZ of mouse pups and adults. Mice were decapitated, brains were removed, placed in ice-cold PBS with antibiotics and sections (1mm thick) containing SVZ were collected. The SVZ was dissected from the lateral wall of the anterior horn of the lateral ventricle. The tissue was digested in StemPro Accutase (ThermoFisher Scientific) solution for 20 min at 37°C. After trituration with P200 and P20 tips, the cell suspension was filtered (50µm filter, BD Biosciences) and plated in NSP medium: DMEM/F12 w/Glutamax and Neurobasal media (1:1) (Gibco) supplemented with 1× B27 (Gibco), 1× N2 (Gibco), 100 U/mL Antibiotic-Antimycotic (Gibco), bFGF (20 ng/ml) (Gibco) and EGF (20 ng/ml) (Gibco) at 37°C with 5% CO₂. Spheres started to appear after approximately 5 days. Cell were passaged every 5-7 days using Accutase.

### Flow cytometry procedure

1. Antibodies: a monoclonal antibody directed to the integrin alpha10 subunit was used. The cells were centrifuged and an aliquot of 100 µL was added per Eppendorf tubes. 1 µg/mL control mouse IgG2a antibody was used as control from 0.2 mg/mL stock.
2. The cells were incubated with primary antibodies for 60 min on ice.
3. The cells were washed twice in 500 µl staining buffer (PBS+/+ with 2% FBS).
4. The secondary antibodies were added and the cells were incubated on ice for 45 minutes.
5. Cells were washed in 500 µL staining buffer 2-3 times.
6. 500 µL staining buffer was added to the final pellet and the cells were resuspended.
7. The cells were analyzed by flow cytometry.

Conclusion: This Example shows that cells isolated from the subventricular zone of adult mouse brain express integrin α10β1, see Figure 1B.

### Example 2: Analysis of co-expression of integrin α10β1 and other markers in mouse brain tissue by immunofluorescence

Fresh frozen mouse brain tissues were embedded in TissueTek (Sakura, Japan). Sections, 10µm (made in a MICROM HM 500 OM Cryostat), were collected on SuperFrost Plus slides (Menzel-Gläser, GmbH). Sections were used for immunolabeling, after post-fixation with acetone (100% at - 20 °C, for 10 min).

Cryo-sections were air-dried in 37°C, for about 20 min. When the sections had reached room temperature they were rinsed twice in PBS (phosphate buffered saline, 0.1 M, pH 7.4) for 5 min. A silicone barrier ("PAP pen") was applied around the sections. The sections were incubated in PBS containing 0.05% (0.1-0.001) Triton X-100 and 1% BSA, for 30 min, at RT and then rinsed in PBS 1 × 2 min. The sections were incubated with a mix of primary antibodies made in different species against different antigens (first evaluated individually for the specificity and optimal working dilution). Incubations were performed for 16-18 hours at 4-8°C, with α10 pAb 1.2 µg/ml (0.6-1.2 µg/ml) (diluted in PBS containing 0.05% Triton X-100 and 1% BSA). For simultaneous fluorescence visualization of two epitopes, the primary and secondary antibodies respectively, were applied as a mixture ("cocktail"). The sections were rinsed in PBS, 1 min followed by 2 × 5 min and incubated with fluorophore conjugated secondary Ab/Abs in a mixture, diluted 1:150, for 30-45min, at RT.

Secondary antibodies for multiple labelling (highly affinity purified, mainly Fab2 fragments) were made in donkey or in goat against rabbit, mouse, or goat IgG's or against chicken IgY (Jackson, USA or Invitrogen, USA) and diluted in PBS containing 1% BSA. For simultaneous fluorescence visualization of two epitopes, the primary and secondary antibodies were applied as a mixture, a "cocktail". The sections were rinsed first in PBS- Triton X100 for 2 min, and then in PBS 1 × 5 min. The sections were incubated in organelle (nuclear) stain DAPI, 0.1 µM, diluted in PBS, for 15 min and rinsed in PBS, 2 × 5 min. The sections were mounted and cover slipped in the "anti-fade solution" ProLong Gold (Invitrogen, USA). The antibodies used in the present study are given in Table 2.

**Table 2. Antibodies used in the present study.**

| **Antigen** | **Host species** | **Supplier** | **Product no.** |
|---|---|---|---|
| Nestin | Rabbit pAb IgG | AbD Serotec | AHP1739 |
| PSA-NCAM | Mouse IgM | Millipore | MAB5324 |
| GFAP | Goat pAb IgG | Abcam | ab53554 |
| PDFGRα | Goat pAb IgG | R&D Systems | AF-307-NA |
| SOX-2 | Mouse mAb | Abcam | ab75485 |
| Integrin alpha10 | Mouse mAb | Xintela AB | mAb365 |
| Integrin | Rabbit pAb | Xintela AB | pAb129 |
| Vimentin | Chicken pAb | Abcam | ab24525 |
| Olig2 | Mouse mAb | Merck | MABN50 |
| CD24 | mouse | BD Horizon | 564521 |
| LeX (CD15/ SSEA-1) | mouse | BioSite | 125609 |
| NeuN | Guinea pig pAb | SYSY | 266004 |
| Iba1 | Goat pAb IgG | Abcam | Ab5076 |

### Analysis:

The analysis was conducted by confocal laser scanning microscopy and epifluorescence.

Specimens were examined in a Zeiss LSM 510 META confocal microscope, utilizing lasers for excitation between 305-633 nm and detection of emission between 420-650 nm. Images were acquired with a 20x/0,8 Plan Apochromate and a 40x/1,3 oil immersion Plan Apochromate objective, with three immunofluorescence channels, one DAPI channels and one bright-field DIC channel.

Z-stacks (no DIC) of consecutive confocal planes were obtained with the 40x/1,3 objective, either with 1024×1024 px frame size (pixel width 0,22 µm), or with "zoom" (scanning a smaller area) and Nyquist optimal sampling frequency (pixel width 0,115 µm) for maximal resolution. Step size between consecutive confocal planes were according to Nyquist optimal sampling frequency (0.48 µm).

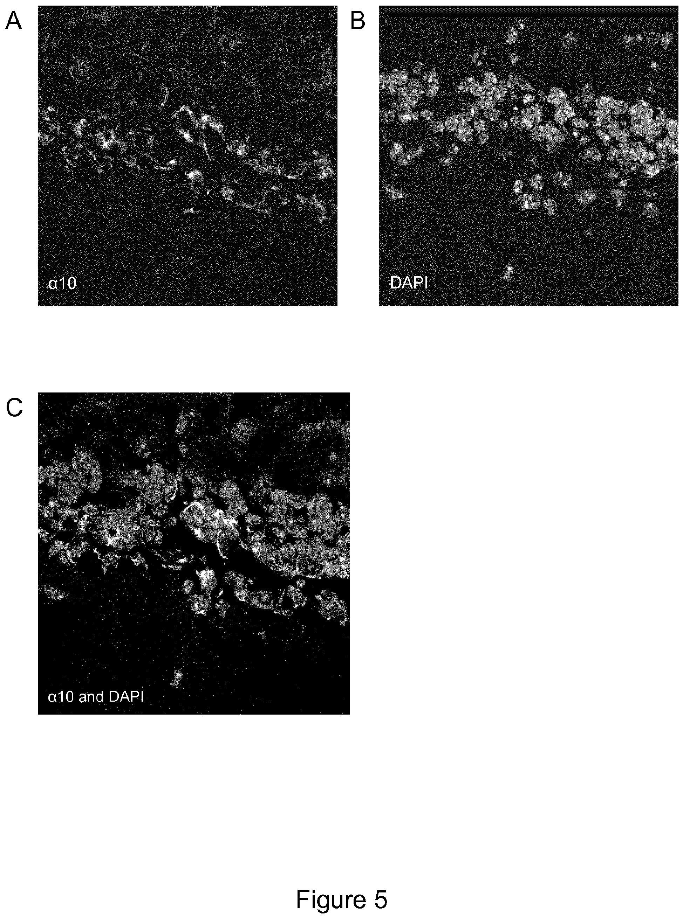

Results: Images from immunofluorescence staining and confocal microscopy indicate expression of integrin α10β1 in the SVZ of adult mouse tissue (Figure 5), expression and co-localization of integrin α10β1 and nestin in the SVZ of adult mouse brain tissue (Figure 6), expression and partial co-localization of integrin α10β1 and PSA-NCAM in the SVZ of adult mouse brain tissue (Figure 7); expression and partial co-localization of integrin α10β1 and GFAP in the SVZ of adult mouse brain tissue (Figure 8), as well as expression and partial co-localization of integrin α10β1 and SOX2 in the SVZ of newborn mouse brain tissue (Figure 9). Partial co-localization of integrin α10β1 and PDFGRα in the SVZ of adult mouse brain tissue was also seen using flow cytometry, as shown in Figure 10.

The images from immunofluorescence staining, confocal microscopy and epifluorescence indicate expression and partial co-localization of integrin α10β1 and SOX2 in the subgranular zone of newborn mouse brain tissue (Figure 11) and expression and partial co-localization of integrin α10β1 and PDFGRα in the meninges of newborn mouse brain tissue (Figure 12).

Conclusion: In this Example, various markers are shown to be expressed and partially co-localized with integrin α10β1 in the SVZ of adult mouse brain tissue.

### Example 3: Differentiation of isolated mouse NSP/NSP cells and gene expression analysis

To confirm specific neural cell lineages, quantitative PCR assay was used to measure gene expression.

### RNA extraction and quantitative PCR

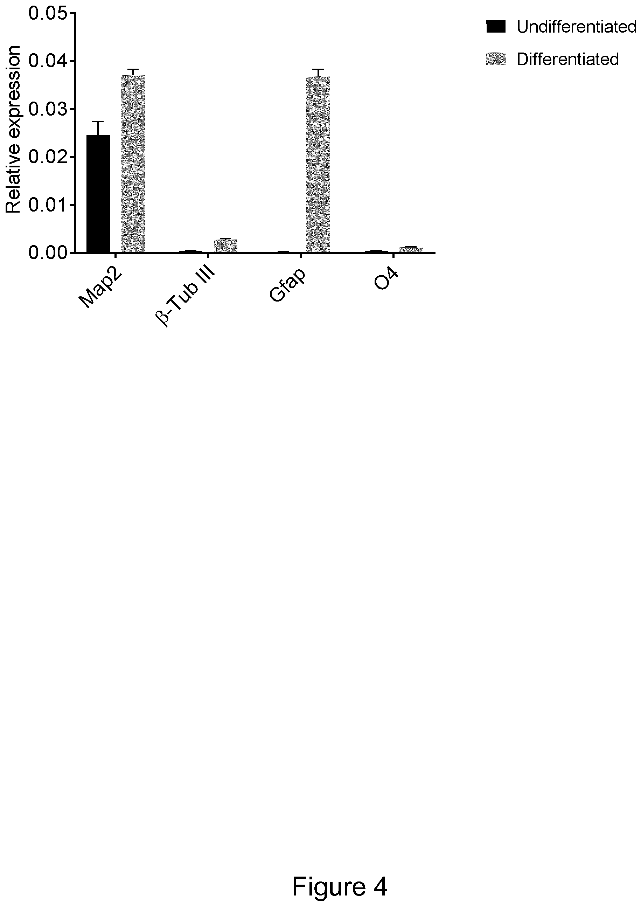

Total RNA was extracted from cells or tissue using an RNeasy Plus micro kit (Qiagen), and then reversed to cDNA using a SuperScript cDNA Synthesis Kit (Life Technologies). For quantitative PCR, TaqMan Gene expression master mix (Life Technologies) and TaqMan probes (ThermoFisher Scientific) were used. Cycle threshold values of target genes were normalized to geometric mean of housekeeping gene Gapdh to get ΔCt. 2 to the power of -ΔCt (2^{-ΔCt}) was calculated for final analysis, see Figure 4. The Taqman probes used for qPCR analysis are given in Table 3.

**Table 3. Taqman probes used for qPCR analysis.**

| Gene Name | Gene Function | TaqMan probe Number |
|---|---|---|
| **GAPDH** | Housekeeping gene | Mm99999915_g1 |
| **Map2** | Microtubule Associated Protein Neuronal marker | Mm00485231_m1 |
| **β III Tub** | Neuronal marker | Mm00727586_s1 |
| **GFAP** | Neural progenitor marker Mature astrocyte | Mm01253033_m1 |
| **O4** | Transcription factor Oligodendrocyte lineage gene | Mm00840140_g1 |

Conclusion: The results showed upregulation of the gene expression for Map2, β-Tubulin III, Gfap and O4 (Figure 4). This indicates the effective differentiation of isolated NSCs/NSPs into neurons, astrocytes and oligodendrocytes.

### Example 4: Integrin alpha10 subunit positive neural stem cells are cultured as neurospheres

Neural stem/progenitor cells were isolated from the SVZ of postnatal and adult mice (C57BI/6). Brains were isolated, the lateral ventricular walls dissected and digested in StemPro Accutase (ThermoFisher Scientific) for 20 min at 37 °C. The tissue was triturated with P200 and p20 tips and the cell suspension filtered (50µm filter, BD Biosciences) and plated in NSP medium (DMEM/F12 w/Glutamax and Neurobasal media (1:1) (Gibco) supplemented with B27 (Gibco), N2 (Gibco), 100 U/mL Antibiotic-Antimycotic (Gibco), bFGF (20 ng/ml) (Gibco) and EGF (20 ng/ml) (Gibco). Cells were passaged every 5 days using Accutase.

Results: Flow cytometry and images from immunofluorescence staining followed by confocal microscopy show that a subpopulation of cells cultured as neurospheres express integrin α10β1 together with other stem cell markers (Figure 2 and Figure 3).

### Example 5: Treatment of stroke in mouse by administering an enriched population of NSCs identified and isolated by detection of integrin alpha10 subunit expression

### Isolation and culture of mouse NSCs

Neural stem cells expressing an integrin alpha10 subunit are isolated from whole mouse brain according to the procedure described in Example 1.

After isolation, the neural stem cells are cultured in neurosphere form as described in Example 4.

Passaging is performed every 4-6 days at a split ratio of 1:3. Normally, the cells at Passage 3 are readily usable for experiments. NSCs at Passage 3 are also used for characterization. Neural stem cell markers CD133 and PDFGRα, as well as integrin α10β1 are examined by flow cytometry.

### Permanent middle cerebral artery occlusion (pMCAO)

pMCAO in mice is surgically generated, for example following the procedure described in Engel et al. (2011).

### Transplantation of different number of the NSCs stable expressing α10β1 cells into stroke afflicted mouse brain

Mice receive single doses of 2.5×10⁶, 5×10⁶ or 10×10⁶ NSCs - α10 cells. Integrin α10β1 positive NSCs are implanted by using magnetic resonance imaging stereotactic techniques to define the target sites surrounding the residual stroke volume.

In conclusion, this study demonstrates that intracerebral stem cell transplant with NSCs- α10 expressing cells for treatment of stoke is generally safe and well tolerated by mice and results in an improved neurological function.

### Isolation and culture of mouse BM-MSCs

Mice aged 4 weeks or 8 weeks are terminated by cervical dislocation and placed in a 100 mm cell culture dish (Becton Dickinson, Franklin Lakes, NJ, USA), where the whole body is soaked in 70% (v/v) ethanol for 2 minutes, and then the mouse is transferred to a new dish. Four claws are dissected at the ankle and carpal joints, and incisions made around the connection between hind limbs and trunk, forelimbs, and trunk. The whole skin is subsequently removed from the hind limbs and forelimbs by pulling toward the cutting site of the claw. Muscles, ligaments, and tendons are carefully disassociated from tibias, femurs, and humeri using microdissecting scissors and surgical scalpel. Tibias, femurs, and humeri are dissected by cutting at the joints, and the bones are transferred onto sterile gauze. Bones are carefully scrubbed to remove the residual soft tissues, and transferred to a 100-mm sterile culture dish with 10 mL complete α-MEM medium on ice. All samples are processed within 30 minutes following animal death to ensure high cell viability. The soft tissues are completely dissociated from the bones to avoid contamination.

In a biosafety cabinet, the bones are washed twice with PBS containing 1% PSN to flush away the blood cells and the residual soft tissues, then bones are transferred into a new 100-mm sterile culture dish with 10 mL complete α-MEM medium. The bone is held with forceps and the two ends excised just below the end of the marrow cavity using microdissecting scissors. A 23-gauge needle attached to a 5 mL syringe is used to draw 5 mL complete α-MEM medium from the dish; then the needle is inserted into the bone cavity. The marrow out is slowly flushed and the bone cavities washed twice again until the bones become pale. All the bone pieces are removed from the dish using forceps, leaving the solid mass in the medium, and the dish is incubated at 37°C in a 5% CO2 incubator for 5 days. In order to obtain enough marrow cells, the bone cavities are flushed repeatedly until the bones appear to be pale.

The initial spindle-shaped cells appear on Day 3 in phase-contrast microscopy, and then culture becomes more confluent and reaches 70-90% confluence within only 2 days. Cells are washed with PBS twice, and digested with 2.5 mL of 0.25% trypsin for 2 minutes at 37°C, then the trypsin neutralised with 7.5 mL complete α-MEM medium. The bottom of the plate is flushed using pipet-aid and the cells transferred to a 15 mL Falcon tube (Becton Dickinson), which is centrifuged at 800g for 5 minutes, and the cells resuspended in a 75 cm² cell culture flask (Corning Inc, Corning, NY, USA) at a split ratio of 1:3.

Passaging are performed every 4-6 days at a split ratio of 1:3. Normally, the cells at Passage 3 contain fewer macrophages and blood cells, and less fat than those at Passages 1 and 2, and are thus readily usable for experiments.

BM-MSCs at Passage 3 are used for characterization. Mesenchymal stem cell markers CD44 and CD90, endothelial cell marker CD31 and haematopoietic marker CD45 are examined by flow cytometry

### Lentiviral Transduction of integrin α10β1 into BM-MSCs

Day 1: Add 1.6 × 10⁴ BM-MSCs cells in fresh media to the number of wells needed for each construct in a 96-well plate. Duplicate or triplicate wells for each lentiviral construct and control should be used. Incubate 18-20 hours at 37°C in a humidified incubator in an atmosphere of 5-7% CO₂.

Day 2: Remove medium from wells. Add 110 µl medium and Hexadimethrine bromide (final concentration 8 µg/ml) to each well. Gently swirl the plate to mix. Add 2-15 µl of α10β1 or control lentiviral particles to appropriate wells. Gently swirl the plate to mix. Incubate 18-20 hours at 37°C in a humidified incubator in an atmosphere of 5-7% CO₂.

Day 3: Remove the medium containing lentiviral particles from wells. Add fresh medium to a volume of 120 µl to each well.

Day 4: Remove medium from wells. Add fresh media containing puromycin.

Day 5 and onwards: Replace medium with fresh puromycin containing medium every 3-4 days until resistant colonies can be identified.

### Permanent middle cerebral artery occlusion (pMCAO)

pMCAO in mice is surgically generated, for example following the procedure described in Engel et al. (2011).

### Transplantation of different number of the BM-MSCs stable overexpressing α10β1 cells into stroke area of mouse brain

Mice receive single doses of 2.5×10⁶, 5×10⁶ or 10×10⁶ BM-MSCs- α10 cells. The NSCs- α10β1 are implanted by using magnetic resonance imaging stereotactic technique to define the target sites surrounding the residual stroke volume.

Conclusion: In conclusion this study demonstrates that intracerebral stem cell transplant with BM-MSCs - α10 expressing cells for treatment of stoke is generally safe and well tolerated by mice and results in an improved neurological function.

### Example 6: Expression of integrin α10β1 in a stroke mouse model

### Stroke model

Procedures were carried out on C57BL/6 mice (25-30g, Charles River, Germany). In brief, permanent focal ischemia model by photothrombosis (PT) was used. Body temperature during surgery was kept at 37 °C. Mice were anesthetized with isoflurane (2% in O2 under spontaneous ventilation). The photosensitive Rose Bengal dye (10 mg/ml, Sigma) was injected intravenously in the tail vein. The skin above the skull was incised. The brain was illuminated through the exposed skull with cold light (Kl 1500 LCD, Schott) and green filter (510 nm) for 20 min at a stereotactically defined position (1 mm laterally and +2/-2 mm anterior/posterior to Bregma). As a control the mouse brain was removed and frozen.

In response to stroke, expression of integrin α10β1 were noticeably increased by day seven (Figure 13). Confocal analysis shows that NeuN expression on neurons was also increased in the stroke area and there was partial colocalization of integrin α10β1 and NeuN (Figure 14 B and C). In addition expression of GFAP, on mature astrocytes, was increased in the stroke area (Figure 14 E and F) and found to colocalize with integrin α10β1.The microglial marker Iba1 was also found within the stroke area (Figure 14 H and I) but not in the control brain tissue (Figure 14G)). However, it did not colocalize with integrin α10β1. This suggests that integrin α10β1 identifies cell types that can be involved in regenerating the brain tissue after stroke.

Conclusion: As a response to stroke, the increased expression of integrin α10β1, alone or along with other markers (NeuN, GFAP and Iba1) can be used as a biomarker to determine stroke area and to predict severity and outcome of the injury in stroke patients.

### References

Bengtsson et al (2005) J Cell Sci. 118(Pt 5):929-36.
Butenschön et al (2016) Stem Cell Research & Therapy 7(11):1-17.
Calaora et al (1996) Neuroscience 73(2): 581-94.
Camper et al (1998) J Biol Chem. 273(32):20383-9.
Camper et al (2001) Cell Tissue Res. 306(1):107-16.
Capaela et al (2002) Neuron 35(5): 865-75.
Capela et al (2006) Developmental Biol. 291(2): 300-313
Chojnacki et al (2011) J. Neurosci. 31(26): 9503-12.
Decimo et al (2012) Curr Pharm 18(13):1755-83.
Decimo et al (2012) Am J Stem Cells. 1(2):92-105.
Doetsch et al (1997) J. Neurosci. 17(13): 5046-61.
Engel et al (2011) J. Vis. Exp. 2011(47): e2423.
Fairbairn et al (2015) World J. Stem Cells 7(1): 11-26.
Farahani et al (2015) Stem Cells Int. 2015.
Flanagan et al (2006) J. Neurosci. Res. 83(5):845-56.
Gage et al (2013) Neuron 80(3): 588-601.
Giachino et al (2009) Stem Cells in Regenerative Medicine, Volume 482 of the series Methods in Molecular Biology pp 143-58.
Guo et al (2012) Nature Protoc. 7(11): 2005-2012.
Hall et al (2006) Stem Cells. 24(9):2078-84.
Jackson et al (2006) Neuron 51(2): 187-99.
Kania et al (2005) Stem Cells 23(6):791-804.
Kim et al (2014) Stem Cell Rev. 10(6):761-71.
Kyöstilä et al (2013) PLoS One. 8(9):e75621.
Lin et al (2015) Brain Res. 1628(Pt B): 327-42.
MacNicol et al (2008) Bioschem. Soc. Trans. 36(Pt 3):528-30.
Engel et al (2011) J Vis Exp. 2011; (47): 2423.
Oliver-De la Cruz et al (2012) Neural Stem Cells and Therapy, book edited by Tao Sun, ISBN 978-953-307-958-5.
Panagiotou et al (2015) Frontiers in Neuroscience Vol. 9, Article 182
Prowse et al (2011) Stem Cell Research 6(1):1-12.
Pruszak et al (2009) Stem Cells 27(12): 2928-40.
Rietze et al (2001) Nature 16;412(6848):736-9.
Steinberg et al (2016) Stroke 47:1817-1824
Varas et al (2007) Stem Cells Dev. 16(6):965-78.
Zhang et al (2006) Cell Research 16: 909-15.

## Claims

1. In vitro use of a marker comprising an integrin alpha10 subunit expressed by a neural cell as a marker for mammalian neural stem cells and mammalian neural progenitor cells, wherein the integrin alpha 10 subunit is expressed on the cell surface of the mammalian neural stem cell and/or neural progenitor cell.

2. An in vitro method for identifying a mammalian neural stem cell and/or a mammalian neural progenitor cell, the method comprising the steps of:
a) detecting expression of an integrin alpha10 subunit by a cell comprised in a sample comprising neural tissue,
b) scoring the integrin alpha10 subunit expression of a), and
c) identifying the mammalian neural stem cell and/or the neural progenitor cell according to the scoring in b), wherein said scoring comprises comparing the measurement with a measurement done in a reference cell population expressing the integrin alpha10 subunit, as well as to a cell population not expressing the integrin alpha10 subunit, and wherein the integrin alpha 10 subunit is expressed on the cell surface of the mammalian neural stem cell and/or neural progenitor cell.

3. An in vitro method for isolating a mammalian neural stem cell and/or a mammalian neural progenitor cell, the method comprising the steps of:
a) detecting expression of an integrin alpha10 subunit by a cell comprised in a sample comprising neural tissue,
b) scoring the integrin alpha10 subunit expression of a), and
c) selecting the mammalian neural stem cell and/or the mammalian neural progenitor cell according to the scoring in b), wherein said scoring comprises comparing the measurement with a measurement done in a reference cell population expressing the integrin alpha10 subunit, as well as to a cell population not expressing the integrin alpha10 subunit, and wherein the integrin alpha 10 subunit is expressed on the cell surface of the mammalian neural stem cell and/or neural progenitor cell,
thereby obtaining an isolated neural stem cell and/or neural progenitor cell.

4. The method according to any one of claims 2 to 3, further comprising a step of contacting the sample with an antibody which specifically binds integrin alpha10 subunit prior to the detection of a).

5. The method according to any one of claims 2 to 4, wherein the neural tissue is derived from brain tissue, such as the neural tissue is adult brain tissue or fetal brain tissue, and wherein the neural tissue is selected from the group consisting of human, canine, equine, bovine, feline, murine, ovine or swine neural tissue.

6. The method according to any one of claims 2 to 5, wherein the neural tissue is derived from the subventricular zone (SVZ) or from the subgranular zone (SGZ) or from the meninges.

7. The method according to any one of claims 2 to 6, wherein the detection of expression of an integrin alpha10 subunit by a cell is determined by measuring integrin alpha10 protein expression or by measuring integrin alpha10 mRNA expression.

8. The method according to any one of claims 2 to 7, wherein the antibody is:
a) a monoclonal antibody, produced by the hybridoma cell line deposited at the Deutsche Sammlung von Mikroorganismen und Zellkulturen GmbH under the accession number DSM ACC2583; or
b) an antibody which competes for binding to the same epitope as the epitope bound by the monoclonal antibody produced by the hybridoma deposited at the Deutsche Sammlung von Mikroorganismen und Zellkulturen GmbH under the accession number DSM ACC2583; or
c) a fragment of a) or b), wherein said fragment is capable of binding specifically to the extracellular I-domain of the integrin alpha10 subunit chain.

9. A method for manufacturing an isolated population of mammalian cells *in vitro* which are enriched for neural stem cells and/or neural progenitor cells relative to a reference population, the method comprising the steps of
a) providing at least a portion of a population of cells comprising a neural stem cell and/or a neural progenitor cell,
b) introducing into the population of cells in a) above a compound identifying an integrin alpha10 subunit expressed by the neural stem cell and/or neural progenitor cell,
c) selecting and isolating, or isolating and selecting, or isolating, or selecting, the neural stem cells and/or the neural progenitor cells from the population of cells in step b) above,
thereby producing a population of cells enriched for neural stem cells and/or neural progenitor cells.

10. An *in vitro* cell culture of undifferentiated mammalian cells, wherein the cells are derived from neural tissue, comprising at least 90% mammalian neural stem cells expressing an integrin alpha10 subunit, wherein the integrin alpha10 subunit is expressed on the cell surface of the undifferentiated mammalian cell and wherein
a) cells in the culture have the capacity to differentiate into neurons and/or oligodendrocytes and/or astrocytes when differentiated in a culture medium substantially free of both serum and a proliferation-inducing growth factor as defined in (b) to produce a cell culture of at least 10% neurons and/or oligodendrocytes and/or astrocytes;
b) the cell culture divides in a culture medium containing a serum replacement such as B27 and at least one proliferation-inducing growth factor;
c) cells in the culture differentiate into neurons and/or oligodendrocytes and/or astrocytes upon withdrawal of both the serum replacement and the proliferation inducing growth factor.

11. A suspension culture of mammalian undifferentiated cells, wherein at least 90% of said cells express an integrin alpha10 subunit, wherein said cells are derived from neural tissue, wherein the undifferentiated mammalian cell is a neural stem cell or a neural progenitor cell, wherein the integrin alpha10 subunit is expressed on the cell surface of the undifferentiated mammalian cell, wherein said cells are formed into cell aggregates, and wherein the cell aggregates are maintained in a culture medium containing a proliferation-inducing growth factor.

12. An in vitro method for determining the characteristics of a damaged or diseased area of the CNS in a patient in need thereof, the method comprising the steps of:
a) administering an anti-integrin alpha10 subunit antibody to a sample of a damaged or diseased area of the CNS of the patient,
b) detecting expression of integrin alpha10 subunit in the sample of the damaged or diseased area of the CNS of the subject,
c) determining characteristics such as location and size of the damaged or diseased area of the CNS.

13. An integrin alpha10 chain subunit marker for mammalian neural stem cells and/or mammalian neural progenitor cells, for use in a method of diagnosing and/or characterizing a disease or damage of the nervous system, wherein the marker is an integrin alpha10 chain subunit expressed on the cell surface of the neural stem cell and/or neural progenitor cells.

14. A composition comprising an isolated population of mammalian neural stem cells and/or mammalian neural progenitor cells for use in a method of treatment of disease or damage of the nervous system wherein the isolated population is enriched for mammalian neural stem cells and/or mammalian neural progenitor cells expressing an integrin alpha10 subunit, and wherein the integrin alpha10 subunit is expressed on the cell surface of the mammalian neural stem cell and/or neural progenitor cell.

15. The method, the marker for use, or the composition for use according to any one of claims 12 to 14, wherein the disease or damage of the nervous system is an injury of the central or peripheral nervous system or a neurodegenerative disease, such as an injury of the nervous system selected from a group consisting of spinal cord injuries (SCI), traumatic brain injuries (TBI), peripheral nerve injuries, stroke and brain cancer; such as a neurodegenerative disease selected from a group consisting of Alzheimer's disease, Parkinson's disease, amyotrophic lateral sclerosis (ALS), Huntington's Disease (HD), multiple sclerosis (MS) and multiple system atrophy.

## Patentansprüche

1. In-vitro-Verwendung eines Markers, der eine Integrin-alpha10-Untereinheit umfasst, die von einer Nervenzelle exprimiert wird, als einen Marker für neurale Säugetierstammzellen und neurale Säugetiervorläuferzellen, wobei die Integrin-alpha10-Untereinheit auf der Zelloberfläche der neuralen Säugetierstammzelle und/oder der neuralen Vorläuferzelle exprimiert wird.

2. In-vitro-Verfahren zum Identifizieren einer neuralen Säugetierstammzelle und/oder einer neuralen Säugetiervorläuferzelle, wobei das Verfahren die folgenden Schritte umfasst:
a) Erkennen einer Expression einer Integrin-alpha10-Untereinheit durch eine Zelle, die in einer Probe umfasst ist, die Nervengewebe umfasst,
b) Bewerten der Expression der Integrin-alpha10-Untereinheit aus a), und
c) Identifizieren der neuralen Säugetierstammzelle und/oder der neuralen Vorläuferzelle gemäß dem Bewerten in b), wobei das Bewerten das Vergleichen der Messung mit einer Messung, die in einer Referenzzellpopulation, die die Integrin-alpha10-Untereinheit exprimiert, erfolgt ist, als auch mit einer Zellpopulation, die die Integrin-alpha10-Untereinheit nicht exprimiert, umfasst, und wobei die Integrin-alpha10-Untereinheit auf der Zelloberfläche der neuralen Säugetierstammzelle und/oder neuralen Vorläuferzelle exprimiert wird.

3. In-vitro-Verfahren zum Isolieren einer neuralen Säugetierstammzelle und/oder einer neuralen Säugetiervorläuferzelle, wobei das Verfahren die folgenden Schritte umfasst:
a) Erkennen einer Expression einer Integrin-alpha10-Untereinheit durch eine Zelle, die in einer Probe umfasst ist, die Nervengewebe umfasst,
b) Bewerten der Expression der Integrin-alpha10-Untereinheit aus a), und
c) Auswählen der neuralen Säugetierstammzelle und/oder der neuralen Säugetiervorläuferzelle gemäß dem Bewerten in b), wobei das Bewerten das Vergleichen der Messung mit einer Messung, die in einer Referenzzellpopulation, die die Integrin-alpha10-Untereinheit exprimiert, erfolgt ist, als auch mit einer Zellpopulation, die die Integrin-alpha10-Untereinheit nicht exprimiert, umfasst, und wobei die Integrin-alpha10-Untereinheit auf der Zelloberfläche der neuralen Säugetierstammzelle und/oder neuralen Vorläuferzelle exprimiert wird,
wodurch eine isolierte neurale Stammzelle und/oder neurale Vorläuferzelle erhalten wird.

4. Verfahren nach einem der Ansprüche 2 bis 3, ferner einen Schritt des Inkontaktbringens der Probe mit einem Antikörper, der spezifisch die Integrin-alpha10-Untereinheit bindet, vor dem Erkennen von a), umfassend.

5. Verfahren nach einem der Ansprüche 2 bis 4, wobei das Nervengewebe von Hirngewebe stammt, das Nervengewebe etwa erwachsenes Hirngewebe oder fetales Hirngewebe ist, und wobei das Nervengewebe aus der Gruppe ausgewählt ist, die aus menschlichem, Hunde-, Pferde-, Rinder-, Katzen-, Mäuse-, Schaf- oder Schweinenervengewebe besteht.

6. Verfahren nach einem der Ansprüche 2 bis 5, wobei das Nervengewebe aus der subventrikulären Zone (SVZ) oder aus der subgranulären Zone (SGZ) oder aus den Hirnhäuten stammt.

7. Verfahren nach einem der Ansprüche 2 bis 6, wobei die Erkennung der Expression einer Integrin-alpha10-Untereinheit durch eine Zelle durch Messen der Integrin-alphalO-Protein-Expression oder durch Messen der Integrin-alpha10-mRNA-Expression ermittelt wird.

8. Verfahren nach einem der Ansprüche 2 bis 7, wobei der Antikörper Folgendes ist:
a) ein monoklonaler Antikörper, der durch die Hybridom-Zelllinie produziert wird, die in der Deutschen Sammlung von Mikroorganismen und Zellkulturen GmbH unter der Zugangsnummer DSM ACC2583 hinterlegt ist; oder
b) ein Antikörper, der um die Bindung an dasselbe Epitop konkurriert wie das Epitop, das von dem monoklonalen Antikörper gebunden wird, der durch das Hybridom produziert wird, das in der Deutschen Sammlung von Mikroorganismen und Zellkulturen GmbH unter der Zugangsnummer DSM ACC2583 hinterlegt ist; oder
c) ein Fragment von a) oder b), wobei das Fragment in der Lage ist, spezifisch an der extrazellulären I-Domäne der Integrin-alpha10-Untereinheit-Kette zu binden.

9. Verfahren zum Herstellen einer isolierten Population von Säugetierzellen in vitro, die in Bezug auf neurale Stammzellen und/oder neurale Vorläuferzellen relativ zu einer Referenzpopulation angereichert ist, wobei das Verfahren die folgenden Schritte umfasst
a) Bereitstellen mindestens eines Teils einer Population von Zellen, die eine neurale Stammzelle und/oder eine neurale Vorläuferzelle umfassen,
b) Einführen in die Population von Zellen in a) oben einer Verbindung, die eine Integrin-alpha10-Untereinheit identifiziert, die von der neuralen Stammzelle und/oder der neuralen Vorläuferzelle exprimiert wird,
c) Auswählen und Isolieren, oder Isolieren und Auswählen, oder Isolieren, oder Auswählen der neuralen Stammzellen und/oder der neuralen Vorläuferzellen aus der Population von Zellen in Schritt b) oben,
dadurch Erzeugen einer Population von Zellen, die in Bezug auf neurale Stammzellen und/oder neurale Vorläuferzellen angereichert sind.

10. In-vitro-Zellkultur von undifferenzierten Säugetierzellen, wobei die Zellen aus Nervengewebe abstammen, die mindestens 90 % neurale Säugetierstamzellen umfasst, die eine Integrin-alpha10-Untereinheit exprimieren, wobei die Integrin-alpha10-Untereinheit auf der Zelloberfläche der undifferenzierten Säugetierzellen exprimiert wird, und wobei
a) Zellen in der Kultur die Fähigkeit aufweisen, sich in Neuronen und/oder Oligodendrozyten und/oder Astrozyten zu differenzieren, wenn sie sich in einem Kulturmedium, das im Wesentlichen frei sowohl von Serum als auch von einem proliferationsinduzierenden Wachstumsfaktor ist, wie in (b) definiert, differenzieren, um eine Zellkultur von mindestens 10 % Neuronen und/oder Oligodendrozyten und/oder Astrozyten zu produzieren;
b) die Zellkultur sich in einem Kulturmedium, das einen Serumersatz wie B27 und mindestens einen proliferationsinduzierenden Wachstumsfaktor enthält, teilt;
c) sich Zellen in der Kultur in Neuronen und/oder Oligodendrozyten und/oder Astrocyten nach Entfernung sowohl des Serumersatzes als auch des proliferationsinduzierenden Wachstumsfaktors, differenzieren.

11. Suspensionskultur von undifferenzierten Säugetierzellen, wobei mindestens 90 % der Zellen eine Integrin-alpha10-Untereinheit exprimieren, wobei die Zellen von Nervengewebe abstammen, wobei die undifferenzierte Säugetierzelle eine neurale Stammzelle oder eine neurale Vorläuferzelle ist, wobei die Integrin-alpha10-Untereinheit auf der Zelloberfläche der undifferenzierten Säugetierzelle exprimiert wird, wobei Zellen zu Zellaggregaten geformt werden, und wobei die Zellaggregate in einem Kulturmedium gehalten werden, das einen proliferationsinduzierenden Wachstumsfaktor enthält.

12. In-vitro-Verfahren zum Ermitteln der Eigenschaften eines geschädigten oder erkrankten Bereichs des ZNS bei einem Patienten, der dies benötigt, wobei das Verfahren die folgenden Schritte umfasst:
a) Verabreichen eines Anti-Integrin-alpha10-Untereinheit-Antikörpers an eine Probe eines geschädigten oder erkrankten Bereichs des ZNS des Patienten,
b) Erkennen einer Expression der Integrin-alpha10-Untereinheit in der Probe des geschädigten oder erkrankten Bereichs des ZNS des Individuums,
c) Ermitteln von Eigenschaften wie Position und Größe des geschädigten oder erkrankten Bereichs des ZNS.

13. Integrin-alphalO-Ketten-Untereinheit-Marker für neurale Säugetierstammzellen und/oder neurale Säugetiervorläuferzellen, zur Verwendung bei einem Verfahren zum Diagnostizieren und/oder Charakterisieren einer Erkrankung oder Schädigung des Nervensystems, wobei der Marker eine Integrin-alphalO-Ketten-Untereinheit ist, die auf der Zelloberfläche der neuralen Stammzelle und/oder der neuralen Vorläuferzellen exprimiert wird.

14. Zusammensetzung, die eine isolierte Population von neuralen Säugetierstammzellen und/oder neuralen Säugetiervorläuferzellen umfasst, zur Verwendung in einem Verfahren der Behandlung einer Erkrankung oder Schädigung des Nervensystems, wobei die isolierte Population in Bezug auf neurale Säugetierstammzellen und/oder neurale Säugetiervorläuferzellen, die eine Integrin-alpha10-Untereinheit exprimieren, angereichert ist, und wobei die Integrin-alpha10-Untereinheit auf der Zelloberfläche der neuralen Säugetierstammzelle und/oder der neuralen Vorläuferzelle exprimiert wird.

15. Verfahren, Marker zur Verwendung oder Zusammensetzung zur Verwendung nach einem der Ansprüche 12 bis 14, wobei die Erkrankung oder Schädigung des Nervensystems eine Verletzung des zentralen oder peripheren Nervensystems ist oder eine neurodegenerative Erkrankung ist, wie etwa eine Verletzung des Nervensystems, die aus einer Gruppe ausgewählt ist, die aus Rückenmarksverletzungen (spinal cord injury - SCI), Schädel-Hirn-Traumata (SHT / traumatic brain injuries - TBI), peripheren Nervenverletzungen, Schlaganfall und Hirntumor besteht; wie etwa eine neurodegenerative Erkrankung, die aus der Gruppe ausgewählt ist, die aus Alzheimer-Krankheit, Parkinson-Krankheit, amyotropher Lateralsklerose (ALS), Chorea Huntington (Huntingston's disease - HD), multipler Sklerose (MS) und Multisystematrophie besteht.

## Revendications

1. Utilisation in vitro d'un marqueur comprenant une sous-unité alpha 10 de l'intégrine exprimée par une cellule neurale en tant que marqueur pour des cellules souches neurales de mammifère et des cellules progénitrices neurales de mammifère, dans laquelle la sous-unité alpha 10 de l'intégrine est exprimée sur la surface cellulaire de la cellule souche neurale de mammifère et/ou de la cellule progénitrice neurale.

2. Procédé in vitro pour identifier une cellule souche neurale de mammifère et/ou une cellule progénitrice neurale de mammifère, le procédé comprenant les étapes de :
a) détection d'une expression d'une sous-unité alpha10 de l'intégrine par une cellule contenue dans un échantillon comprenant un tissu neural,
b) évaluation de l'expression de la sous-unité alpha10 de l'intégrine de l'étape a), et
c) identification de la cellule souche neurale de mammifère et/ou de la cellule progénitrice neurale conformément à l'évaluation de l'étape b), dans lequel ladite évaluation comprend la comparaison de la mesure avec une mesure effectuée dans une population de cellules de référence exprimant la sous-unité alpha10 de l'intégrine, ainsi que dans une population de cellules n'exprimant pas la sous-unité alpha10 de l'intégrine, et dans lequel la sous-unité alpha10 de l'intégrine est exprimée sur la surface cellulaire de la cellule souche neurale de mammifère et/ou de la cellule progénitrice neurale.

3. Procédé in vitro pour isoler une cellule souche neurale de mammifère et/ou une cellule progénitrice neurale de mammifère, le procédé comprenant les étapes de :
a) détection d'une expression d'une sous-unité alpha10 de l'intégrine par une cellule contenue dans un échantillon comprenant un tissu neural,
b) évaluation de l'expression de la sous-unité alpha10 de l'intégrine de l'étape a), et
c) sélection de la cellule souche neurale de mammifère et/ou de la cellule progénitrice neurale de mammifère conformément à l'évaluation de l'étape b), dans lequel ladite évaluation comprend la comparaison de la mesure avec une mesure effectuée dans une population de cellules de référence exprimant la sous-unité alpha10 de l'intégrine, ainsi que dans une population de cellules n'exprimant pas la sous-unité alpha10 de l'intégrine, et dans lequel la sous-unité alpha10 de l'intégrine est exprimée sur la surface cellulaire de la cellule souche neurale de mammifère et/ou de la cellule progénitrice neurale,
obtenant ainsi une cellule souche neurale et/ou une cellule progénitrice neurale isolée(s).

4. Procédé selon l'une quelconque des revendications 2 et 3, comprenant en outre une étape de mise en contact de l'échantillon avec un anticorps qui se lie spécifiquement à une sous-unité alpha10 de l'intégrine avant la détection de l'étape a).

5. Procédé selon l'une quelconque des revendications 2 à 4, dans lequel le tissu neural est issu de tissu cérébral, par exemple un tissu neural adulte ou un tissu neural fœtal, et dans lequel le tissu neural est choisi dans le groupe constitué de tissu neural humain, canin, équin, bovin, félin, murin, ovin ou porcin.

6. Procédé selon l'une quelconque des revendications 2 à 5, dans lequel le tissu neural est issu de la zone sous-ventriculaire (SVZ) ou de la zone sous-granulaire (SGZ) ou des méninges.

7. Procédé selon l'une quelconque des revendications 2 à 6, dans lequel la détection de l'expression d'une sous-unité alpha10 de l'intégrine par une cellule est déterminée en mesurant l'expression de la protéine alpha10 de l'intégrine ou en mesurant l'expression de l'ARNm alpha10 de l'intégrine.

8. Procédé selon l'une quelconque des revendications 2 à 7, dans lequel l'anticorps est :
a) un anticorps monoclonal, produit par la lignée cellulaire d'hybridome déposée auprès de Deutsche Sammlung von Mikroorganismen und Zellkulturen GmbH sous le numéro d'accès DSM ACC2583 ; ou
b) un anticorps qui entre en compétition pour se lier au même épitope que l'épitope lié par l'anticorps monoclonal produit par l'hybridome déposé auprès de Deutsche Sammlung von Mikroorganismen und Zellkulturen GmbH sous le numéro d'accès DSM ACC2583 ; ou
c) un fragment de a) ou de b), dans lequel ledit fragment peut se lier spécifiquement au domaine I extracellulaire de la chaîne de sous-unité alpha10 de l'intégrine.

9. Procédé de fabrication d'une population isolée de cellules de mammifère *in vitro* qui sont enrichies en cellules souches neurales et/ou en cellules progénitrices neurales par rapport à une population de référence, le procédé comprenant les étapes de
a) fourniture d'au moins une partie d'une population de cellules comprenant une cellule souche neurale et/ou une cellule progénitrice neurale,
b) introduction, dans la population de cellules de l'étape a) ci-dessus, d'un composé identifiant une sous-unité alpha10 de l'intégrine exprimée par la cellule souche neurale et/ou la cellule progénitrice neurale,
c) sélection et isolement, ou isolement et sélection, ou isolement, ou sélection, des cellules souches neurales et/ou des cellules progénitrices neurales à partir de la population de cellules de l'étape b) ci-dessus,
produisant ainsi une population de cellules enrichies en cellules souches neurales et/ou en cellules progénitrices neurales.

10. Culture cellulaire *in vitro* de cellules indifférenciées de mammifère, dans laquelle les cellules sont issues de tissu neural, comprenant au moins 90 % de cellules souches neurales de mammifère exprimant une sous-unité alpha10 de l'intégrine, dans laquelle la sous-unité alpha10 de l'intégrine est exprimée sur la surface cellulaire de la cellule de mammifère indifférenciée et dans laquelle
a) des cellules dans la culture ont la capacité de se différencier en neurones et/ou en oligodendrocytes et/ou en astrocytes lorsqu'elles sont différenciées dans un milieu de culture sensiblement exempt à la fois de sérum et d'un facteur de croissance induisant la prolifération tel que défini à l'étape (b) pour produire une culture cellulaire d'au moins 10 % de neurones et/ou d'oligodendrocytes et/ou d'astrocytes ;
b) la culture cellulaire se divise dans un milieu de culture contenant un substitut de sérum, comme le B27, et au moins un facteur de croissance induisant la prolifération ;
c) des cellules dans la culture se différencient en neurones et/ou en oligodendrocytes et/ou en astrocytes lors du retrait à la fois du substitut de sérum et du facteur de croissance induisant la prolifération.

11. Culture en suspension de cellules indifférenciées de mammifère, dans laquelle au moins 90 % desdites cellules expriment une sous-unité alpha10 de l'intégrine, dans laquelle lesdites cellules sont issues de tissu neural, dans laquelle la cellule indifférenciée de mammifère est une cellule souche neurale ou une cellule progénitrice neurale, dans laquelle la sous-unité alpha10 de l'intégrine est exprimée sur la surface cellulaire de la cellule indifférenciée de mammifère, dans laquelle lesdites cellules sont formées en agrégats de cellules, et dans laquelle les agrégats de cellules sont maintenus dans un milieu de culture contenant un facteur de croissance induisant la prolifération.

12. Procédé in vitro permettant de déterminer les caractéristiques d'une zone lésée ou malade du SNC chez un patient qui en a besoin, le procédé comprenant les étapes de :
a) administration d'un anticorps de la sous-unité alpha10 anti-intégrine à un échantillon d'une zone lésée ou malade du SNC du patient,
b) détection de l'expression de la sous-unité alpha10 de l'intégrine dans l'échantillon de la zone lésée ou malade du SNC du sujet,
c) détermination de caractéristiques telles que l'emplacement et la taille de la zone lésée ou malade du SNC.

13. Marqueur de sous-unité de chaîne alpha10 de l'intégrine pour des cellules souches neurales de mammifère et/ou des cellules progénitrices neurales de mammifère, à utiliser dans un procédé de diagnostic et/ou de caractérisation d'une maladie ou d'une lésion du système nerveux, dans lequel le marqueur est une sous-unité de chaîne alpha10 de l'intégrine exprimée sur la surface cellulaire de la cellule souche neurale et/ou des cellules progénitrices neurales.

14. Composition comprenant une population de cellules isolée souches neurales de mammifère et/ou de cellules progénitrices neurales de mammifère, à utiliser dans un procédé de traitement d'une maladie ou d'une lésion du système nerveux, dans laquelle la population isolée est enrichie en cellules souches neurales de mammifère et/ou en cellules progénitrices neurales de mammifère exprimant une sous-unité alpha10 de l'intégrine, et dans laquelle la sous-unité alpha10 de l'intégrine est exprimée sur la surface cellulaire de la cellule souche neurale de mammifère et/ou de la cellule progénitrice neurale.

15. Procédé, marqueur à utiliser, ou composition à utiliser selon l'une quelconque des revendications 12 à 14, dans lequel la maladie ou la lésion du système nerveux est une lésion du système nerveux central ou périphérique ou une maladie neurodégénérative, telle qu'une lésion du système nerveux choisie dans un groupe constitué des lésions de la moelle épinière (LME), des traumatismes crânio-cérébraux (TCC), des lésions nerveuses périphériques, d'un accident vasculaire cérébral et d'un cancer du cerveau ; tel(s) ou telle(s) qu'une maladie neurodégénérative choisie dans un groupe constitué de la maladie d'Alzheimer, la maladie de Parkinson, la sclérose latérale amyotrophique (SLA), la maladie de Huntington (MH), la sclérose en plaques (SP) et l'atrophie multisystématisée.
